(19) **Europäisches Patentamt / European Patent Office / Office européen des brevets**

(11) **EP 4 482 618 B1**

(12) **EUROPEAN PATENT SPECIFICATION**

(45) Date of publication and mention of the grant of the patent:
**31.12.2025 Bulletin 2026/01**

(21) Application number: **23712105.8**

(22) Date of filing: **21.02.2023**

(51) International Patent Classification (IPC):
**B01J 21/06** (2006.01)  **C10G 3/00** (2006.01)

(52) Cooperative Patent Classification (CPC):
(C-Sets available)
**C07C 45/29; B01J 21/066; B01J 23/72; B01J 37/0201; B01J 37/031; C10G 3/44; C10G 3/54;** B01J 35/70; C10G 2400/08      (Cont.)

(86) International application number:
**PCT/IT2023/050054**

(87) International publication number:
**WO 2023/157041 (24.08.2023 Gazette 2023/34)**

(54) **PROCESS FOR THE PRODUCTION OF MIXTURES USABLE AS JET FUEL OR JET FUEL PRECURSORS STARTING FROM C2-C4 ALCOHOLS**

VERFAHREN ZUR HERSTELLUNG VON GEMISCHEN, DIE AUS C2-C4-ALKOHOLEN ALS FAHRZEUGKRAFTSTOFF ODER VORLÄUFER FÜR FAHRZEUGKRAFTSTOFFE VERWENDBAR SIND

PROCÉDÉ DE FABRICATION DE MÉLANGES UTILISABLES COMME FUEL JET OU PRÉCURSEURS DE FUEL JET A PARTIR D'ALCOOLS C2-C4

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **21.02.2022 IT 202200003209**

(43) Date of publication of application:
**01.01.2025 Bulletin 2025/01**

(73) Proprietors:
• **Alma Mater Studiorum - Università di Bologna**
  **40126 Bologna (BO) (IT)**
• **GST Gestioni Servizi Tecnologie S.r.l.**
  **00148 Roma (RM) (IT)**
• **IG Operation and Maintenance S.p.A.**
  **00071 Pomezia (RM) (IT)**

(72) Inventors:
• **TABANELLI, Tommaso**
  **40126 Bologna (BO) (IT)**
• **CAVANI, Fabrizio**
  **40126 Bologna (BO) (IT)**
• **BALESTRA, Giulia**
  **40126 Bologna (BO) (IT)**
• **GAGLIARDI, Anna**
  **40126 Bologna (BO) (IT)**
• **BERRUTI, Massimo**
  **00148 Roma (RM) (IT)**

(74) Representative: **Ceccarelli, Ilaria et al**
  **Barzanò & Zanardo S.p.A.**
  **Via Piemonte, 26**
  **00187 Roma (IT)**

(56) References cited:
**WO-A1-2015/120524      US-A1- 2014 171 696
US-A1- 2018 215 692**

• **HAMELINCK ET AL: "Production of FT transportation fuels from biomass; technical options, process analysis and optimisation, and development potential", ENERGY, ELSEVIER, AMSTERDAM, NL, vol. 29, no. 11, 1 September 2004 (2004-09-01), pages 1743 - 1771, XP005229162, ISSN: 0360-5442, DOI: 10.1016/J.ENERGY.2004.01.002**

**(Cont. next page)**

EP 4 482 618 B1

- **SATO A G ET AL: "Effect of the ZrO2phase on the structure and behavior of supported Cu catalysts for ethanol conversion", JOURNAL OF CATALYSIS, ACADEMIC PRESS, DULUTH, MN, US, vol. 307, 2 August 2013 (2013-08-02), pages 1 - 17, XP028736363, ISSN: 0021-9517, DOI: 10.1016/J.JCAT.2013.06.022**
- **N. SCOTTI: "Copper-zirconia catalysts: powerful multifunctional catalytic tools to approach sustainable processes", CATALYSTS, vol. 10, no. 2, 1 February 2020 (2020-02-01), pages 168, XP002807536**

(52) Cooperative Patent Classification (CPC): (Cont.)

C-Sets
**C07C 45/29, C07C 47/06**

**Description**

The present invention relates to a process for the production of mixtures usable as a jet fuel or jet fuel precursors starting from C2-C4 alcohols, such as ethanol and butanol.

In particular, the invention relates to a process for the production of a mixture comprising compounds having a number of carbon atoms compatible with the range of carbon atoms typical of the jet fuel, in particular comprising compounds having a number of carbon atoms greater than or equal to 6, said mixture being produced starting from C2-C4 alcohols, or mixtures thereof, wherein metallic copper on a zirconia-based support is used as a catalyst.

It is known that the production of alternative fuels from biomasses is a topic of enormous interest for both academic and industrial research. In particular, while the production of biodiesel starting from vegetable oils or animal fats has already been widely investigated in the literature and applied industrially, the production of mixtures, usable as a jet fuel, starting from ethanol (obtainable from biomasses by fermentation) constitutes a new and highly promising, as well as competitive, field of research.

Although the ASTM standard (Standard Specification for Aviation Turbine Fuels, Designation: D1655 - 22; https://www.astm.org/d1655-22.html) does not define a specific composition for jet fuels, the properties required for a jet fuel are obtained from mixtures containing hydrocarbons with a certain cut. In particular, in the literature it is shown that the wide-cut jet fuel (jet fuel with a wide distillation range) is characterised by a distribution of a number of carbon atoms between about 5 and 15 carbon atoms (https://www.chevron.com/- /media/chevron/operations/documents/aviation-tech-review.pdf).

US patent US10221119B2 relates to the conversion of ethanol to $C_5$+ ketones in a single catalyst bed. In the process of the above-mentioned patent a mixed metal oxide catalyst, comprising $ZrO_2$-ZnO with 0.05% by weight of Pd as a promoter, or a $CuO$-$MgO$-$Al_2O_3$ catalyst is used.

The same authors of the above-mentioned patent have subsequently also published a scientific work in the international journal Angewandte Chemie (S. Subramaniam, M. F. Guo, T. Bathena, M. Gray, X. Zhang, A. Martinez, L. Kovarik, K. A. Goulas and K. K. Ramasamy, Angewandte Chemie International Edition, 2020, 59, 14550-14557). This article includes the selective synthesis of aliphatic ketones, in particular the linear ones, with chain lengths between $C_5$ and $C_{11}$ starting from ethanol. These ketones are considered key intermediates for the synthesis of lubricants and alternative fuels starting from renewables (including jet fuels). In particular, the catalyst used consists of a small percentage of Pd (even only 0.1%) supported on a solid mixture of ZnO and $ZrO_2$. Catalytic tests were performed using a fixed bed tubular reactor in down-flow mode, condensing the heavy products in a cold trap and analysing the light/incondensable ones via an online GC. Most of the tests were carried out at temperatures between 300 and 400°C, pressures between 14.7 and 300 psi (in practice between 10 and 20 bar) and with a weight hourly space velocity (WHSV) equal to 0.15 $h^{-1}$. Using the Pd-$ZnO$-$ZrO_2$ catalyst with 0.1% by weight of Pd, ketone yields higher than 70% have been achieved, obtaining a stable catalyst for more than 2000 working hours. The key points emphasised by the authors for the success of such procedure are two: from the mechanistic point of view the fact of being able to obtain acetone as a reaction intermediate which leads to the formation of pentanone ($C_5$ ketone) by means of an aldol condensation reaction with acetaldehyde; furthermore, the *in situ* formation of an alloy between Zn and Pd is essential to create the active sites on the catalyst, capable of giving selectivity in ketones, as well as high activity and stability to the catalytic system. ZnO and $ZrO_2$ are classic catalysts for the Lebedev reaction, i.e. the conversion of ethanol to butadiene, through the mechanism of aldol condensation. This, together with the mechanism proposed by the same authors, suggests that the proposed process promotes the traditional aldol condensation with the formation of ketones, in particular pentanone, obtained after the aldol condensation between acetone and acetaldehyde (mechanism illustrated by the same authors).

Alternative strategies for converting ethanol into mixtures applicable to the fuel field are the following:

i) the Vertimass process, described in patent US10815163B2, wherein acid catalysts (zeolites) modified with other metals are used in order to promote the condensations and dehydration of alcohols, in order to obtain mixtures of hydrocarbons (starting from $C_5$) and aromatic compounds;
ii) the process described in patent US10745330B2 (*Method of converting ethanol to higher alcohols*), wherein catalysts based on copper oxides (in very low amounts), magnesium and aluminium ($CuO$-$MgO$-$Al_2O_3$, Cu at maximum 0.25% by weight) are used for obtaining mixtures of higher alcohols from ethanol; in this method the reactions are carried out under hydrogen pressure (about 13-14 bar), in order to limit the catalyst deactivation and maximise the selectivity in the production of higher aliphatic alcohols through aldol condensations (Guerbet reaction), and at temperatures between 275 and 350°C.

Therefore, more generally, the known processes aimed at obtaining higher molecular weight compounds from ethanol mainly follow three strategies:

- using acid catalysts (zeolites) in order to favour condensations and dehydrations, forming olefins, hydrocarbons and aromatic compounds (such as the above-mentioned Vertimass process);

- using basic catalysts functionalised with metals capable of providing redox characteristics: catalysts capable of promoting aldol condensations with the formation mainly of longer chain alcohols and aldehydes (as for example described in the above-mentioned patent US10745330B2);
- using of acid-base catalysts functionalised with metals capable of providing redox characteristics: the latter strategy can lead to the selective formation of ketones, promoting the aldol condensation reactions (as for example described in the above-mentioned patent US10221119B2 and S. Subramaniam, et al, 2020).

In light of the above, there is a clear need to provide new processes for producing alternative bio-fuels starting from ethanol (or bioethanol) or other alcohols able to optimise the conversion of the starting alcohol and for obtaining high molecular weight molecules (>C5).

US2018/0215692 discloses a method for upgrading alcohols such as ethanol to higher products. It is therefore an object of the present invention a process according to claim 1. The solution according to the present invention fits in this context, which proposes to provide an alternative process for the valorisation of C2-C4 alcohols through the production of a complex mixture of products usable, directly or after hydrogenation of the aldehydes and unsaturated compounds, as a jet fuel. As stated above, the mixture according to the invention comprises compounds having a number of carbon atoms compatible with the range of carbon atoms typical of the jet fuel. In particular, the mixture according to the invention preferably comprises compounds having a number of carbon atoms higher than or equal to 6, which can belong to one or more classes of compounds including for example ketones, esters, alcohols, aldehydes, aliphatic hydrocarbons and aromatic compounds. According to the present invention, preferably, the selectivity of the $\geq$C6 fraction of said mixture is at least 10%, more preferably reaching values of at least 20% during the first 6 hours of reaction, even more preferably between 20% and 40%, such as between 25% and 40%.

The $\geq$C6 compounds contained in the mixture according to the present invention can be employed as jet fuel or they can be used as direct jet fuel precursors if subjected to suitable treatments, such as hydrogenation and/or dehydration.

In particular, according to the present invention, a polyfunctional catalyst has been identified which is able to catalyse different reactions in the same reactor.

More specifically, the catalyst according to the present invention, for example Cu/t-ZrO$_2$ with 5% by weight of Cu, is able to effectively catalyse the dehydrogenation of alcohols to the corresponding aldehydes (in particular, ethanol to acetaldehyde and butanol to butyraldehyde), promoting the dehydrogenative coupling (DHC) reaction and forming the corresponding esters (for example ethyl acetate and butyl butyrate, but also asymmetric esters such as butyl acetate or others). The catalyst employed in the process according to the invention is also able to effectively promote the ketonisation reaction of these esters, making it possible to obtain symmetrical and asymmetrical ketones, in particular in the range between C$_3$ and C$_{11}$. The excess of acetaldehyde produced using the process according to the invention also makes it possible to favour aldol condensations leading to the formation of medium-long chain (C$_6$-C$_{14}$) branched alcohols and ketones. The formation of acetone and acetaldehyde according to the process of the invention also makes it possible the formation of cyclic compounds, in particular alkylated cyclohexanones and cyclohexanols and aromatic (phenolic) compounds, whose presence is accepted in jet fuel blends up to a maximum of 25% and can modulate/modify the properties of the jet fuel itself.

The process according to the present invention is conceptually significantly different from all the alternative alcohol upgrading strategies used for obtaining jet fuels, which are shown in the scientific and patent literature.

In particular, unlike the known processes, the process according to the invention is a "one-pot" process, i.e. it operates on a single catalyst, in a single reaction condition, for the upgrading of C2-C4 alcohols, such as ethanol or butanol, to jet fuel blends. Despite the existence of processes which can produce mixtures of products characterised by the prevalence of a family of compounds (aromatics and olefins in the Vertimass process, ketones in the process described in US10221119B2 and in S. Subramaniam, et al, 2020), the process according to the invention is able to catalyse a series of cascade reactions which lead to the obtainment of a mixture containing all the aforementioned components and other classes of compounds in amounts which can be modulated on the basis of the composition of the catalyst itself and of the operating conditions.

Therefore, the process according to the present invention advantageously provides the exploitation of a series of cascade reactions in order to maximise the alcohol conversion and the obtainment of high molecular weight molecules ($\geq$C6). To do this, a catalyst was used (for example Cu/ZrO$_2$ with 5% by weight of Cu), known for being able to effectively catalyse the dehydrogenative coupling reaction of alcohols with the corresponding esters especially in the liquid phase and under reaction conditions different from those provided by the process of the present invention. In particular, the reaction, under the conditions normally used in literature, is selective for the formation of esters. According to the present invention, instead, the catalyst is used in such conditions as to favour the ketonisation reaction of the esters obtained in order to obtain the corresponding ketones having a number of carbon atoms equal to the sum of the carbon atoms of the starting esters minus one. Through this cascade mechanism, the aldehydes obtained as intermediates can also react via aldol condensation leading to the formation of longer chain (even branched) alcohols or ketones. These, together with cyclic compounds such as cyclohexanones, cyclohexanols and phenols (formed by the reactions of aldol condensations taking place on acetone, the shortest of the ketones) can advantageously make it possible to directly obtain a mixture comprising compounds having a number of carbon atoms compatible with that presented by the compounds contained in the jet fuel (after separation of the lighter component and the unreacted acetaldehyde).

The use of a relatively simple and cheap catalytic system according to the present invention, such as for example $Cu/ZrO_2$ with 5% by weight of Cu, makes it possible to favour a series of cascade reactions, in this way shifting the equilibrium of the various reactions toward the formation of products with ever greater molecular weight. The obtained mixture, thanks to its particular composition, can be employed as a jet fuel or direct jet fuel precursor, for example by means of possible preliminary operations such as the removal by distillation of the lighter compounds (in particular the unconverted acetaldehyde) and a mild hydrogenation process in order to stabilise the mixture by converting the aldehydes and unsaturations into the corresponding alcohols and saturated chains.

Furthermore, according to the present invention the use of $ZrO_2$ having a different crystalline structure or the insertion, in the structure of $ZrO_2$, of elements increasing its basic character, such as for example lanthanum, advantageously increase the catalyst's performance. In particular, the use of lanthanum as a support not only helps to promote ketonisation (as known) but contributes to modify the equilibrium of all the other reactions leading to a very low production of aromatics, improving the stability of the material and limiting the deactivation.

Regarding the $Cu/ZrO_2$ catalyst according to the invention, although copper was already known to be an active metal in the dehydrogenation of alcohols and zirconia and lanthanum were already known to catalyse the ketonisation reaction of acids or esters, under reaction conditions similar to those of the process of the invention, the process according to the invention operates under conditions such that the catalyst is surprisingly able to promote all the aforementioned "one pot" reactions in an extremely efficient manner, i.e. operating on a single catalyst, in a single reaction condition, showing an effect which is synergistic (not simply additive) and not easily predictable.

It is therefore a specific object of the present invention a process for the production of a mixture comprising compounds having a number of carbon atoms $\geq 6$, usable as a jet fuel or jet fuel precursor starting from one or more alcohols having from 2 (C2) to 4 (C4) carbon atoms, wherein said one or more alcohols are introduced into a continuously operated fixed bed catalyst reactor, in an amount from 1 to 100 mol%, in the vapour phase, and at a temperature ranging from 200°C to 450°C, preferably 300°C;

said catalyst comprising metallic copper on a zirconia-based support ($Cu/ZrO_2$), said metallic copper being in a percentage by weight ranging from 0.5% to 25% of the total weight of the catalyst.

The process according to the present invention can be carried out at a pressure ranging from atmospheric pressure to 10 bar, preferably from atmospheric pressure to 5 bar, more preferably from atmospheric pressure to 2 bar, even more preferably at atmospheric pressure.

According to the present invention, said one or more alcohols can be selected from ethanol, propanol, in particular 1-propanol or 2-propanol, butanol such as for example 1-butanol and 2-butanol, and mixtures thereof, preferably ethanol.

According to the present invention, said one or more alcohols, for example ethanol, is introduced into said reactor in an amount from 1 to 40 mol%, preferably from 5 to 40 mol%, more preferably from 1 to 25 mol%, for example from 5 to 25 mol%, even more preferably in an amount of 10 mol%.

According to the present invention, the temperature can range from 250°C to 340°C and is preferably around 300°C.

According to the present invention, said metallic copper can be in a percentage by weight from 0.5% to 25%, preferably from 1% to 10%, more preferably in a percentage by weight of 5%.

According to an embodiment of the present invention, said one or more alcohols, such as for example ethanol, can be introduced into said reactor, diluted in an inert gas flow. In particular, according to the present invention, said inert gas flow may consist of helium, nitrogen, argon and/or another inert gaseous component, such as for example water vapour or carbon dioxide.

According to the process of the present invention, said zirconia can be tetragonal zirconia ($t-ZrO_2$) or monoclinic zirconia ($m-ZrO_2$).

According to an embodiment of the present invention, said catalyst comprising metallic copper on a zirconia-based support does not comprise zinc (Zn).

According to an embodiment of the process according to the present invention, said zirconia-based support can comprise a lanthanide included in the structure of said zirconia, such as for example lanthanum (La-Zr-O) or yttrium. In particular, the Zr:lanthanide atomic ratio can range from 50:1 to 1:1, preferably from 20:1 to 2:1, more preferably 5:1.

Since the basic sites are fundamental for ketonisation, aldol condensation and stability of the catalyst, the use of a monoclinic $ZrO_2$ having a different crystalline structure or the insertion, in the $ZrO_2$ structure, of elements increasing its basic character such as for example lanthanum, increase the catalyst's performance. In particular, the use of lanthanum as a support not only helps to promote ketonisation (as known) but contributes to modify the equilibrium of all the other reactions leading to a very low production of aromatics, improving the stability of the material and limiting the deactivation.

Furthermore, by modifying the support it is possible to decrease the selectivity in acetaldehyde and aromatic compounds.

According to the present invention, said catalyst can be selected from a catalyst comprising metallic copper on a tetragonal zirconia-based (Cu/t-ZrO$_2$) support wherein the metallic copper is present in an amount of 5% by weight of the total weight of the catalyst; a catalyst comprising metallic copper on a tetragonal zirconia-based (Cu/t-ZrO$_2$) support wherein the metallic copper is present in an amount of 1% by weight of the total weight of the catalyst; a catalyst comprising metallic copper on a tetragonal zirconia-based (Cu/t-ZrO$_2$) support wherein the metallic copper is present in an amount of 10% by weight of the total weight of the catalyst; a catalyst comprising metallic copper on a monoclinic zirconia-based (Cu/m-ZrO$_2$) support wherein the metallic copper is present in an amount of 5% by weight of the total weight of the catalyst; a catalyst comprising metallic copper on a zirconia-based support comprising lanthanum included in the structure of said zirconia (Cu/La-Zr-O) wherein the metallic copper is present in an amount of 5% by weight of the total weight of the catalyst.

According to the process of the present invention, said one or more alcohols, such as for example ethanol, is placed in contact with said catalyst for a period of time $\tau$, wherein $\tau$(s) = catalyst volume (mL)/total volumetric flow (mL/s), ranging from 1.5 to 5 seconds, preferably 1.5 to 3 seconds, more preferably 1.85 to 2.53 seconds, still more preferably 2 seconds.

According to an embodiment of the process according to the invention, hydrogen can be fed, preferably at atmospheric pressure, together with said one or more alcohols or, when said one or more alcohols are introduced into the reactor in an inert gas flow, together with said inert gas flow. Therefore, hydrogen can be used in place of the inert gas carrier or in mixtures with it.

According to the present invention, the inert gas (for example nitrogen or helium):H$_2$ ratio can vary from 1:10 to 10:1, for example it can be 1:1.

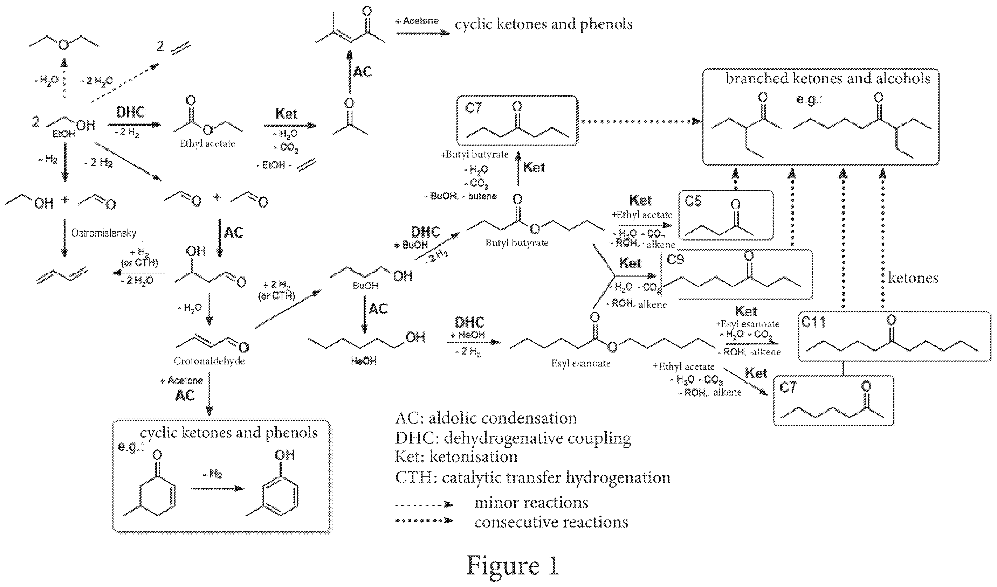

The hydrogen co-feeding seems to perform a modulating function in the dehydrogenation reaction of alcohols to acetaldehyde, a key intermediate in the reactions of the process of the invention, whose excessive concentration on the surface of the catalyst, however, could favour the formation of heavy residues promoting catalyst deactivation. The result obtained in example 12 below shows the ability of the catalyst according to the invention to activate hydrogen at atmospheric pressure by shifting and modulating the dehydrogenation equilibrium. These effects lead to a greater stability of the catalyst (thus suggesting a lower formation of carbonaceous material capable of blocking its active sites), and a lower presence of unsaturated compounds. This hypothesis is supported, for example, by the absence of crotonaldehyde among the main products (example 12), whose absence can explain the lower selectivity in aromatic compounds which derive in part from the condensation of crotonaldehyde itself with acetone (Figure 1).

By inserting a hydrogen co-feeding, the deactivation phenomenon clearly improves, with a constant selectivity of interest in the C$_6$+ fraction for 13 h of reaction. At the same time, there is almost no production of aromatics.

According to the present invention, said catalyst can be in the form of a powder. In particular, said powder can be aggregated into pellets, for example into pellets of size from 20 to 40 mesh.

According to the process of the invention, said mixture can include esters, in particular linear and branched esters, alcohols, in particular branched and linear alcohols, ketones, in particular branched, linear and cyclic ketones, aldehydes, aliphatic hydrocarbons, such as for example alkanes and olefins, and aromatic compounds, in particular phenolic compounds.

Furthermore, the process according to the present invention can further comprise a hydrogenation step of the aldehydes and unsaturated compounds and, optionally, a dehydration step.

A further object not according to the present invention is a plant for the production of a mixture comprising compounds having a number of carbon atoms ≥6, usable as jet fuel or as a jet fuel precursor, starting from one or more alcohols having from 2 to 4 atoms of carbon, such as for example ethanol, butanol or mixtures thereof, according to the process as defined above, said plant comprising

a continuously operating fixed bed catalytic reactor;
a vaporiser placed upstream of said reactor and adapted to bring said one or more alcohols into the vapour phase;
an inlet of said one or more alcohols in the vapour phase and of inert gas toward the inside of said reactor, said inlet being connected to said vaporiser and an inert gas source;
an outlet of said mixture which can be connected to a condenser for the recovery of the products of interest and for subsequent separations.

Said fixed bed catalytic reactor can be a fixed bed tubular reactor in down-flow mode.

Considering the complexity of the mixture of obtainable products, for the control and analysis of the composition of the obtained mixture, a trap at the outlet of the plant, preferably at 25°C so as to favour the condensation of all the heavy products (>C4), and an online gas chromatograph equipped with two thermal conductivity detectors (TCD), placed in series with the trap to analyse the incondensables and the lighter compounds, can be provided.

The present invention will now be described, for illustrative but not limiting purposes, according to a preferred embodiment thereof, with particular reference to the examples and figures of the attached drawings, wherein:

- **Figure 1** shows a simplified reaction scheme of upgrading reactions of ethanol to jet fuel blends according to the process of the present invention;
- **Figure 2** shows the catalytic test results under the following reaction conditions: t-ZrO$_2$, 10 mol% EtOH in He, contact time 2 s, 300°C, in the absence of copper;
- **Figure 3** shows the catalytic test results under the following reaction conditions: m-ZrO$_2$, 10 mol% EtOH in He, contact time 2 s, 300°C, in the absence of copper;
- **Figure 4** shows the catalytic test results under the following reaction conditions: La-Zr-O, 10 mol% EtOH in He, contact time 2 s, 300°C, in the absence of copper;
- **Figure 5** shows the catalytic test results under the following reaction conditions: Cu/t-ZrO$_2$ with 5% by weight of Cu, 10 mol% EtOH in He, contact time 1 s, 300°C;
- **Figure 6** shows the catalytic test results under the following reaction conditions: Cu/t-ZrO$_2$ with 5% by weight of Cu, 10 mol% EtOH in He, contact time 2 s, 300°C;
- **Figure 7** shows the ≥C$_6$ fraction composition expressed as a molar fraction in percentage of the total ≥C$_6$ fraction; reaction conditions: Cu/t-ZrO$_2$ catalyst with 5% by weight of Cu, 10 mol% EtOH in He, contact time 2 s, 300°C;
- **Figure 8** shows the catalytic test results under the following reaction conditions: Cu/t-ZrO$_2$ with 5% by weight of Cu, 10 mol% EtOH in He, contact time 3 s, 300°C;
- **Figure 9** shows the catalytic test results under the following reaction conditions: Cu/t-ZrO$_2$ with 1% by weight of Cu, 10 mol% EtOH in He, contact time 2 s, 300°C;
- **Figure 10** shows the catalytic test results under the following reaction conditions: Cu/t-ZrO$_2$ with 10% by weight of Cu, 10 mol% EtOH in He, contact time 2 s, 300°C;
- **Figure 11** shows the catalytic test results under the following reaction conditions: Cu/m-ZrO$_2$ with 5% by weight of Cu, 10 mol% EtOH in He, contact time 2 s, 300°C;
- **Figure 12** shows the ≥C$_6$ fraction composition expressed as a molar fraction in percentage of the total ≥C$_6$ fraction; reaction conditions: Cu/m-ZrO$_2$ catalyst with 5% by weight of Cu, 10 mol% EtOH in He, contact time 2 s, 300°C;
- **Figure 13** shows the catalytic test results under the following reaction conditions: Cu/La-Zr-O with 5% by weight of Cu, 10 mol% EtOH in He, contact time 2 s, 300°C;
- **Figure 14** shows the ≥C$_6$ fraction composition expressed as a molar fraction in percentage of the total ≥C$_6$ fraction; reaction conditions: Cu/La-Zr-O catalyst with 5% by weight of Cu, 10 mol% EtOH in He, contact time 2 s, 300°C;
- **Figure 15** shows the catalytic test results under the following reaction conditions: Cu/t-ZrO$_2$ with 5% by weight of Cu, 10 mol% EtOH in He:H$_2$ mixture in a 1:1 ratio, contact time 2 s, 300°C;
- **Figure 16** shows the ≥C$_6$ fraction composition expressed as a molar fraction in percentage of the total ≥C$_6$ fraction; reaction conditions: Cu/t-ZrO$_2$ catalyst with 5% by weight of Cu, 10 mol% EtOH in He:H$_2$ mixture in a 1:1 ratio, contact time 2 s, 300°C;
- **Figure 17** shows the catalytic test results under the following reaction conditions: Cu/t-ZrO$_2$ with 5% by weight of Cu, 10 mol% 1-butanol in He, contact time 2 s, 300°C;
- **Figure 18** shows the ≥C$_6$ fraction composition expressed as a molar fraction in percentage of the total ≥C$_6$ fraction; reaction conditions: Cu/t-ZrO$_2$ with 5% by weight of Cu, 10 mol% 1-butanol in He, contact time 2 s, 300°C.

With particular reference to the scheme shown in Figure 1, without wanting to be limited by theory, it is believed that the reaction of the process according to the present invention comprises the following steps:

- dehydrogenation of ethanol to acetaldehyde;
- aldol condensation of acetaldehyde to C$_4$ aldol;
- direct dehydrogenative coupling of alcohols with corresponding esters (aldehydes as reactive intermediates);
- ketonisation of esters to ketones characterised by a number of carbon atoms equal to the sum of the carbon atoms of the acid part of the two esters minus one (CO$_2$ co-production);
- consecutive aldol condensations of acetone and acetaldehyde to make cyclic ketones and/or alcohols;
- dehydrogenation of cyclic to aromatic compounds;
- possible dehydration or decarboxylation of compounds containing oxygen and aliphatic hydrocarbons (alkanes and olefins).

The process according to the present invention exploits the ability of the Cu/ZrO$_2$-based catalyst to develop large quantities of acetaldehyde and to simultaneously promote the dehydrogenative coupling reaction of alcohols to give esters. These, thanks to the high reactivity of zirconia for the ketonisation reaction, are effectively converted to (symmetrical and not symmetrical) ketones.

In particular, the mixture obtained according to the process of the invention consists of (linear and branched) esters, (branched and linear) alcohols, (branched, linear and cyclic) ketones, aldehydes, aliphatic hydrocarbons (alkanes and olefins) and aromatic compounds (especially phenolic compounds).

By way of example, a mixture obtained, according to the process of the invention, from one of the preliminary tests has proved to be promising for applications such as Jet fuel A and A1. In this regard, some information obtained by comparing the properties of the mixture obtained according to the process of the invention with the specific ASTM D1655 mixture are shown below:

Aromatics: 36% against a maximum of 25% for A and A-1;
Olefins: not specified in ASTM Std;
Density: 865 against 775 - 840 for A and A-1;
Gross Heat of Combustion: 37.7 MJ/kg against a minimum of 42.8 MJ/kg for A and A-1;
Naphthalene: 0.93% against a maximum of 3% for A and A-1;
Flash point: 34°C against a minimum of 38°C for A and A1;
Sulfur: 0% (not analysed) against a maximum of 0.3%;
Freezing point: -36.5°C against a maximum of -40°C for A and a maximum of -47°C for A1.

## EXAMPLES

*Reference examples*: catalytic tests on zirconia-based supports in the absence of copper.

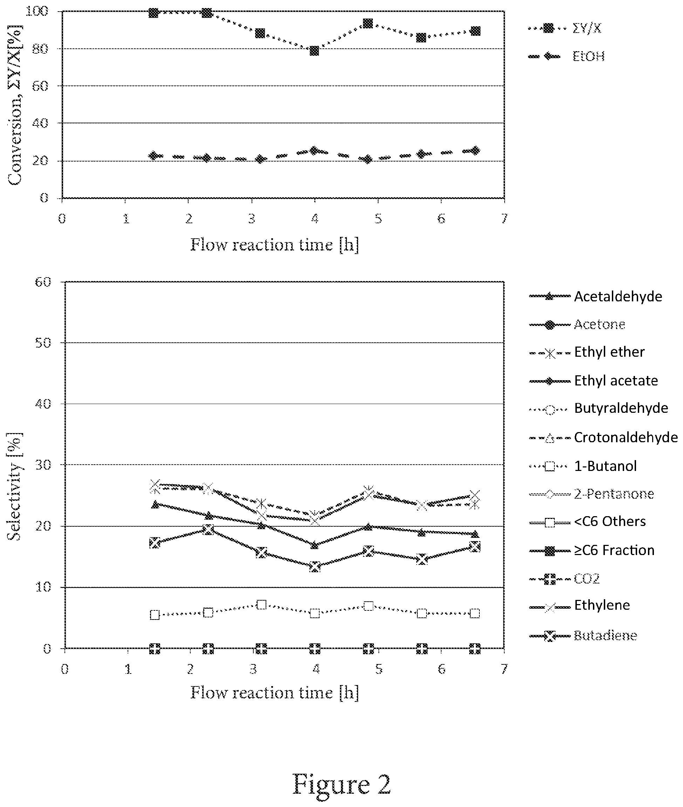

As mentioned above, the process according to the invention operates under conditions such that the catalyst is surprisingly capable of promoting all the "one-pot" reactions in an extremely efficient manner, showing a synergistic and not easily predictable effect. In order to show the improving and inventive effect of the method according to the present invention, the ethanol decomposition tests, carried out on the various supports used in examples 1-14 in the absence of copper, are hereby proposed as reference examples. In particular, in the reference examples it is shown the catalytic activity of the individual supports in the ethanol upgrading reaction under the same reaction conditions used for the copper-containing catalysts shown in the following examples. By observing the results obtained in the reference example (shown in Figures 2-4) it is possible to notice how, regardless of the support taken into consideration, the conversion of ethanol is around 20% with preferential formation of compounds bound in acid catalysis (ethylene, diethyl ether) and products from the Guerbet and Lebedev reactions (acetaldehyde, butadiene and butanol). Heavy compounds (>C6) are formed in trace amounts only on the monoclinic zirconia support.

By comparing the results obtained in the reference example with the results obtained in examples 1-14, it is possible to notice how the addition of copper as active metal on these catalytically active supports produces both an unpredictable increase in the conversion of ethanol and a radical change of the distribution of the obtainable products, making it possible to obtain complex mixtures usable as jet fuel.

*Reference 01*: Catalytic test results under conditions: $t$-$ZrO_2$, 300°C, $\tau$=2 s

The tetragonal $ZrO_2$ support ($t$-$ZrO_2$) was synthesised by precipitation at controlled temperature and pH starting from an aqueous solution of $ZrO(NO_3)_2$. In particular, the solution of the metal precursor (0.3 mol/l) was dripped, under vigorous stirring, into an ammonia aqueous solution (5 mol/l at 25°C) promoting the precipitation of a white solid (hydroxide derivative of the precursor). Subsequently, the solution containing the solid was subjected to a digestion process lasting 24 h at 100°C, maintaining the pH between 9 and 11 by progressively and continuously adding a concentrated ammonia aqueous solution (28% w/w). The sample was then filtered, dried and calcined at 500°C for 12 h at a ramp rate of 5°C/min. This procedure allows the synthesis of a $ZrO_2$ characterised by a tetragonal crystalline phase.

The catalytic test was carried out as shown in example 2, modifying the overall volumetric flow and the amount of loaded catalyst so as to obtain tau = 2 s. The results are shown in Figure 2.

*Reference 02*: Catalytic test results under conditions: $m$-$ZrO_2$, 300°C, $\tau$=2 s

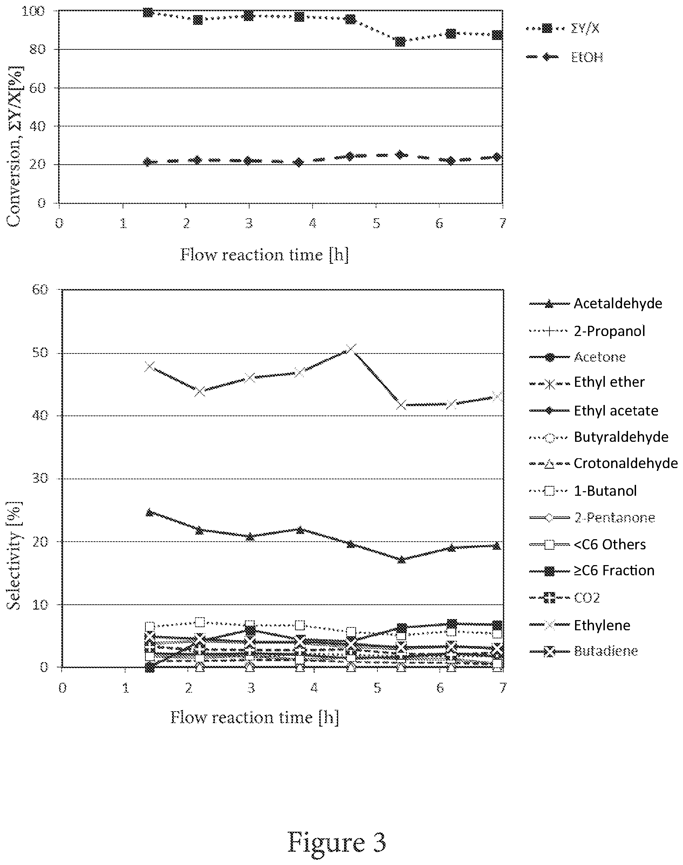

The $ZrO_2$ support was obtained by hydrothermal synthesis in an autoclave at 140°C for 20 h at autogenous pressure starting from an aqueous solution of $ZrO(NO_3)_2$ and urea. The product is washed several times with ethanol, then it is dried and calcined at 450°C for 3 h, at a ramp rate of 5°C/min. In this way zirconia in the monoclinic crystalline phase is obtained. The catalytic test was carried out as shown in example 2, modifying the overall volumetric flow and the amount of loaded catalyst so as to obtain tau = 2 s. The results are shown in Figure 3.

*Reference 03*: Catalytic test results under conditions: La-Zr-O, 300°C, $\tau$=2 s

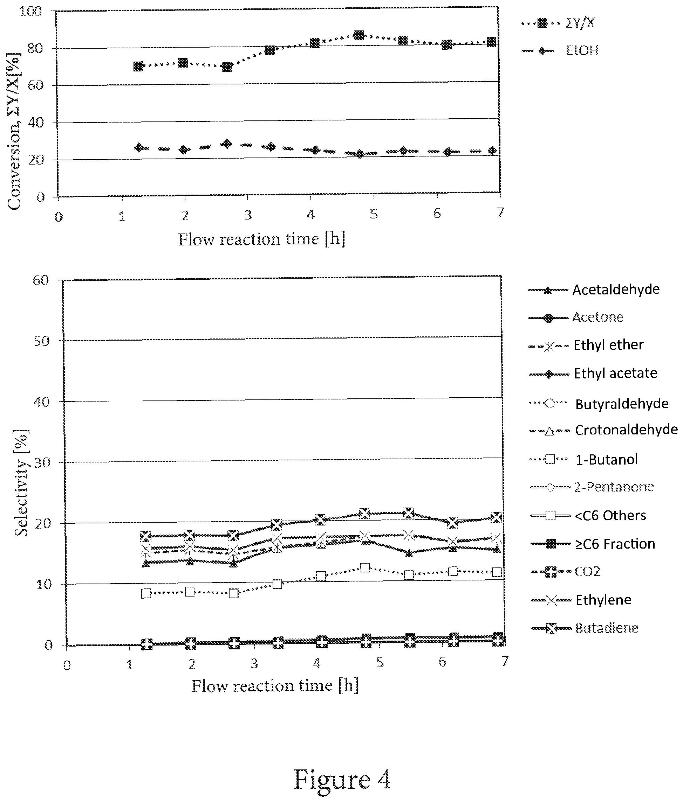

The La-Zr-O support was synthesised by precipitation in a basic environment at 25°C starting from an aqueous solution of $ZrO(NO_3)_2*2H_2O$ and $La(NO_3)_3*6H_2O$ such that the atomic ratio La:Zr in the final material was 0.19 and the total cation concentration was 0.3 mol/l. The suspension was subjected to a digestion of 72 h at 100°C and at controlled pH 10-12 by continuously adding a concentrated ammonia aqueous solution (28% w/w). The sample was then filtered, dried and calcined at 450°C for 12 h at a ramp rate of 5°C/min. The catalytic test was carried out as shown in example 2, modifying the overall volumetric flow and the amount of loaded catalyst so as to obtain tau = 2 s. The results are shown in Figure 4.

**Example 1**: Synthesis of the Cu/t-ZrO$_2$ catalyst with 5% by weight of Cu.

The tetragonal ZrO$_2$ support (t-ZrO$_2$) was synthesised by precipitation at controlled temperature and pH starting from an aqueous solution of ZrO(NO$_3$)$_2$. In particular, the solution of the metal precursor (0.3 mol/l) was dripped, under vigorous stirring, into an aqueous solution of ammonia (5 mol/l at 25°C) promoting the precipitation of a white solid (hydroxide derivative of the precursor). Subsequently, the solution containing the solid was subjected to a digestion process lasting 24 h at 100°C, maintaining the pH between 9 and 11 by progressively and continuously adding a concentrated ammonia aqueous solution (28% w/w). The sample was then filtered, dried and calcined at 500°C for 12 h at a ramp rate of 5°C/min. This procedure allows the synthesis of a ZrO$_2$ characterised by a tetragonal crystalline phase. The Cu was deposited on the support using the incipient wetness impregnation (IWI) method, by dissolving the amount of precursor (Cu(NO$_3$)$_2$) necessary to obtain a copper load of 5% by weight of the final catalyst in a volume of distilled water equal to the volume of the pores of the support. This solution is added drop by drop and evenly distributed on the support until it reaches the slurry point (filling of the pores). After which the sample is dried in a furnace and calcined at 500°C for 5 h at a ramp rate of 2°C/min.

The powder thus obtained is subjected to a pelletising process so as to obtain particles of a defined size (20-40 mesh).

A suitable amount of material is loaded into the plant operating continuously in the vapour phase as described in example 2 and subjected to a reduction process in a hydrogen flow at 350°C for 3 hours so as to obtain the desired catalyst.

**Example 2**: "Standard" catalytic test procedure and results under the conditions: Cu/t-ZrO$_2$ with 5% by weight of Cu, 300°C, $\tau$=1 s

The catalytic tests were carried out in a tubular quartz reactor, operating continuously, in the vapour phase and at atmospheric pressure. In particular, the catalyst, obtained as described in example 1, is loaded into the reactor in the form of pellets (20-40 mesh), so as to be inserted into the isothermal area of the reactor supported by a suitable porous quartz septum. The reactor operates in "down-flow" mode. The organic reagent, ethanol (EtOH), is fed as a liquid through a high precision infusion pump (KPS 100 Syringe Pump) into a vaporisation section consisting of a stainless-steel line maintained at a temperature of 135°C in order to favour an instantaneous vaporisation of the reagent and an adequate mixing with the inert current (He) entering the plant. The vaporisation zone is in fact connected to the quartz reactor (length 600 mm, internal diameter 11 mm) in such a way as to guarantee the tightness of the whole system. The reactor, containing the catalyst, is placed inside a tube furnace. The volume of loaded catalyst, the flows of the syringe for feeding EtOH and the flow of He, controlled by a dedicated Mass Flow meter, are suitably selected so as to have a contact time ($\tau$= Catalyst volume/total volumetric flow) on the catalyst equal to 1 second at the chosen reaction temperature (300°C) with a percentage of ethanol being fed equal to 10 mol% (90 mol% He).

Even the lines of the plant downstream the reactor are insulated and, through the use of electric resistances controlled by thermocouples, heated at a temperature of 250°C. The mixture leaving the reactor is passed through suitable traps maintained at 25°C so as to favour the condensation of only the heavier products. The light products and the non-condensables are analysed using an online Agilent 6890A GC equipped with two different chromatographic columns and two TCD detectors (carrier: He). In particular: a PLOT-Q column (30 m x 0.32 mm x 20 $\mu$m) for the analysis of compounds such as CO$_2$, ethylene and butadiene; a DB-1701 column (30 m x 0.53 mm x 1 $\mu$m) for the analysis of alcohols, aldehydes, esters, ketones and other non-condensed products into the trap.

The traps are changed approximately every hour and the condensed products contained inside are recovered, diluted in methanol and added with octane as an internal standard for the analyses. The solutions thus obtained are analysed by gas chromatograph coupled with a mass spectrometer (GC-MS, Agilent 6890N coupled with mass spectrometer Agilent Technologies 5973 Inert) equipped with an HP-5ms capillary column (30 m x 250 $\mu$m x 0.25 $\mu$m). The details of the analysis method are as follows: injected volume 0.5 $\mu$L, injector $\tau$: 280°C, split ratio: 50:1, carrier: He 1 ml/min, temperature ramp: isotherm at 40°C for 7 minutes then ramp rate at 5°C/min up to 100°C and ramp rate at 10°C/min up to 250°C, temperature then maintained for 2 minutes.

Conversions (X), yields (Y), selectivity (S) and carbon balance were calculated as follows:

$$\chi\% = \frac{n_{EtOH}^{in} - n_{EtOH}^{out}}{n_{EtOH}^{in}} \times 100$$

$$Y_p\% = \frac{C_{atom}^{p} \cdot n_p^{out}}{C_{atom}^{EtOH} \cdot n_{EtOH}^{in}} \times 100$$

$$S_p\% = \frac{Y_p}{\chi} \times 100$$

$$Carbon\ balance = \frac{\sum_i Y_{p_i}}{\chi} \times 100$$

"n" being equal to the number of moles of the particular product "p" (or of the ethanol, EtOH) and "C" being the number of carbon atoms contained in the particular product "p" (or in the ethanol, EtOH).

In particular, the 40 most abundant and recurring chemical compounds in the mixtures exiting the reactor were selected and calibrated (by means of calibration straight lines with suitable commercial standards, where available, or with suitable isomers) in order to quantify their yields with a suitable response factor. Unless otherwise specified in the figures, the calibrated compounds and the remaining obtained products were grouped into three groups:

"Light others" ($C_3$-$C_5$): GC-MS retention times from 0 to 6 minutes;
"Mid others" ($C_6$-$C_8$): from 6 to 16.5 minutes;
"Heavy others" (>Cs): from 16.5 minutes to the end of the analysis.

The response factor of these compounds was estimated on the basis of the known response factors for the most similar molecules exiting in the same range of retention times and by averaging the number of carbon atoms of the molecules in the same range. The yields of these compounds were then calculated as follows:

$$Y_{LIGHT\ OTHERS}\% = \frac{4 \cdot mol_p^{out}}{C_{atom}^{EtOH} \cdot mol_{EtOH}^{in}} \times 100$$

$$Y_{MID\ OTHERS}\% = \frac{7 \cdot mol_p^{out}}{C_{atom}^{EtOH} \cdot mol_{EtOH}^{in}} \times 100$$

$$Y_{HEAVY\ OTHERS}\% = \frac{10 \cdot mol_p^{out}}{C_{atom}^{EtOH} \cdot mol_{EtOH}^{in}} \times 100$$

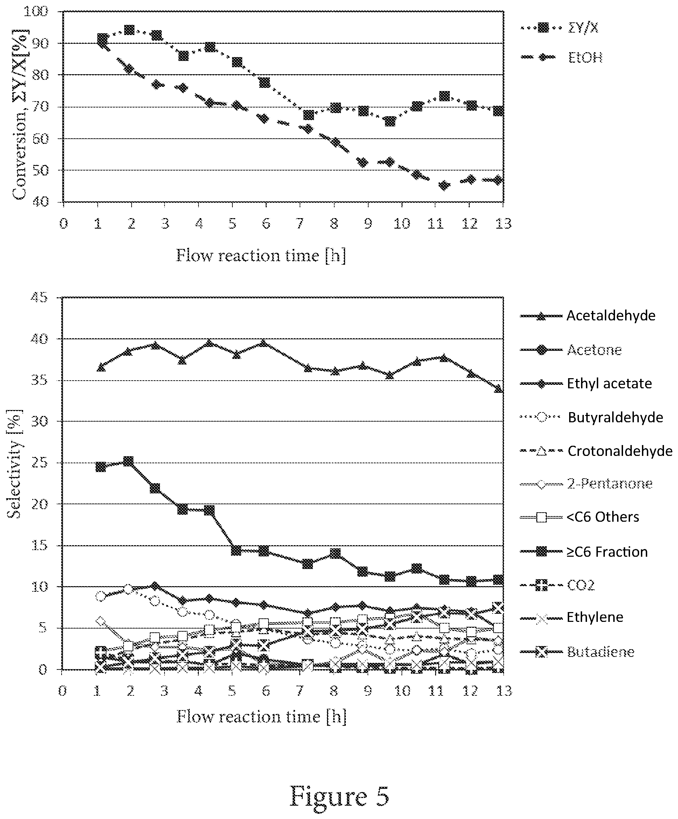

The results of the catalytic test are shown in Figure 5.

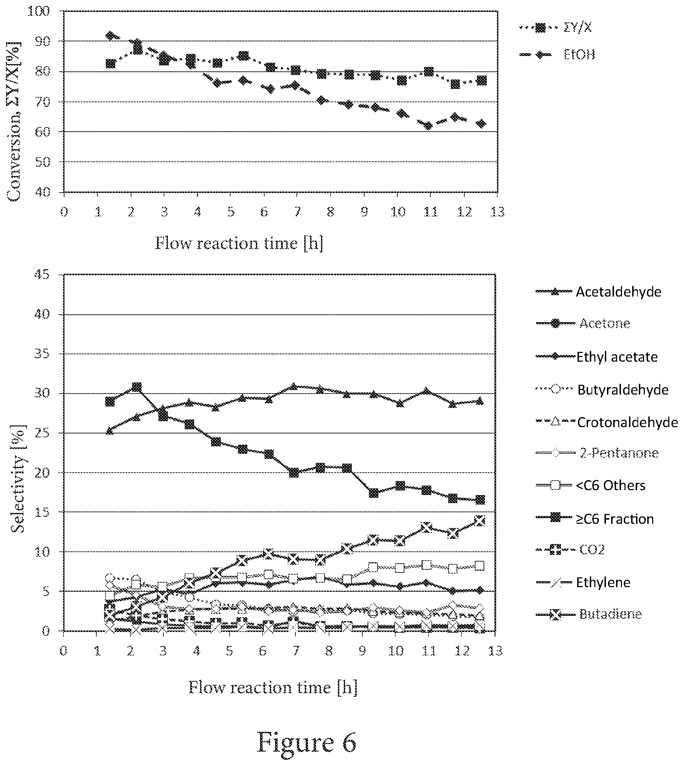

It can be observed how the conversion of ethanol undergoes a more sudden drop as a function of the reaction time in the flow and the selectivity in the >C6 fraction generally remains lower than in Figure 6, example 3 (same conditions but tau=2). This leads to a yield in the >C6 fraction equal to 5% under conditions of tau=1 s after 12 hours of reaction, while this yield is equal to 11% (more than double) under conditions of tau = 2 s.

**Example 3:** *catalytic test under the conditions: Cu/t-ZrO$_2$ with 5% by weight of Cu, 300°C, $\tau$=2 s*

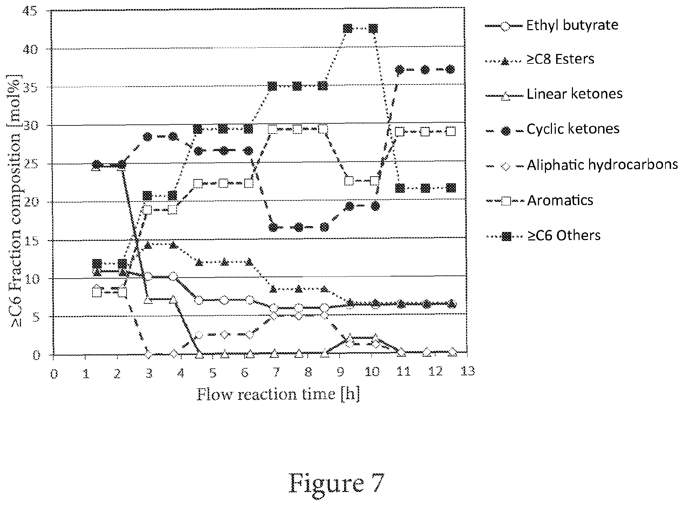

The catalyst used, Cu/t-ZrO$_2$ with 5% by weight of Cu, was synthesised as in example 1. The catalytic test was carried out as shown in example 2, modifying the overall volumetric flow and the amount of loaded catalyst so as to obtain tau = 2 s. The results are shown in Figure 6. The ≥C6 fraction composition is shown in Figure 7.

Under the above optimised conditions, the use of the Cu/t-ZrO$_2$ catalyst with 5% by weight of Cu (5% by weight of metallic copper supported on tetragonal zirconia) made it possible to obtain ethanol conversions equal to 90% during the first two hours, with the product distribution shown in the following tables, which summarise the results shown in Figures 6 and 7. The percentages correspond to the $S_P$ selectivities expressed as the ratio between product yield $Y_P$ and ethanol conversion $X_{EtOH}$; $SP=Y_P/X_{EtOH}$. The yields are normalised on the carbon atoms of the various products. The "$<C_6$ others" fraction brings together light minority components, of which *n*-butanol is the main one.

In particular, Table 1 shows the mixture composition exiting the plant after two hours of reaction, comparing it with that averaged over the first six hours of reaction.

**Table 1:** *reaction mixture composition exiting the plant. Reaction conditions: 300°C, contact time: 2 s, 10 mol% EtOH in He, Cu/t-ZrO$_2$ with 5% by weight of Cu. Ethanol conversion: 90% after 2 h, 83% averaged over the first 6 h.*

| Compound | Selectivity after 2 h | Selectivity averaged over 6 h |
|---|---|---|
| Acetaldehyde | 27% | 28% |
| Acetone | 2% | 1% |
| Ethyl acetate | 4% | 5% |
| Butyraldehyde | 7% | 5% |
| Crotonaldehyde | 2% | 3% |
| 2-pentanone | 5% | 3% |
| Butadiene | 3% | 6% |
| $CO_2$ | 2% | 1% |
| $<C_6$ others | 6% | 6% |
| $\geq C_6$ fraction | 31% | 26% |

The "$\geq$Ce fraction" is further divided into the following classes of compounds (Table 2). The composition is expressed as a molar fraction in percentage of the total $\geq C_6$ fraction ($T_{eb}>120°C$) obtained by removing the $<C_6$ fraction with $T_{eb}<120°C$. The $\geq C_6$ others fraction consists mainly of higher alcohols and aldehydes, together with other minority compounds.

**Table 2:** *details about the "$\geq$C6 fraction" composition. Reaction conditions: 300°C, contact time: 2 s, 10 mol% EtOH in He, Cu/t-ZrO$_2$ with 5% by weight of Cu.*

| Compound | Molar fraction % after 2 h | Molar fraction % averaged over 6 h |
|---|---|---|
| Ethyl butyrate | 11% | 9% |
| $\geq C_8$ esters | 11% | 12% |
| Linear ketones | 25% | 11% |
| Cyclic ketones | 25% | 27% |
| Aliphatic hydrocarbons | 8% | 4% |
| Aromatics | 8% | 16% |
| $\geq C_6$ others | 12% | 21% |

**Example 4:** *catalytic test under the conditions: Cu/t-ZrO$_2$ with 5% by weight of Cu, 300°C, $\tau$=3 s*

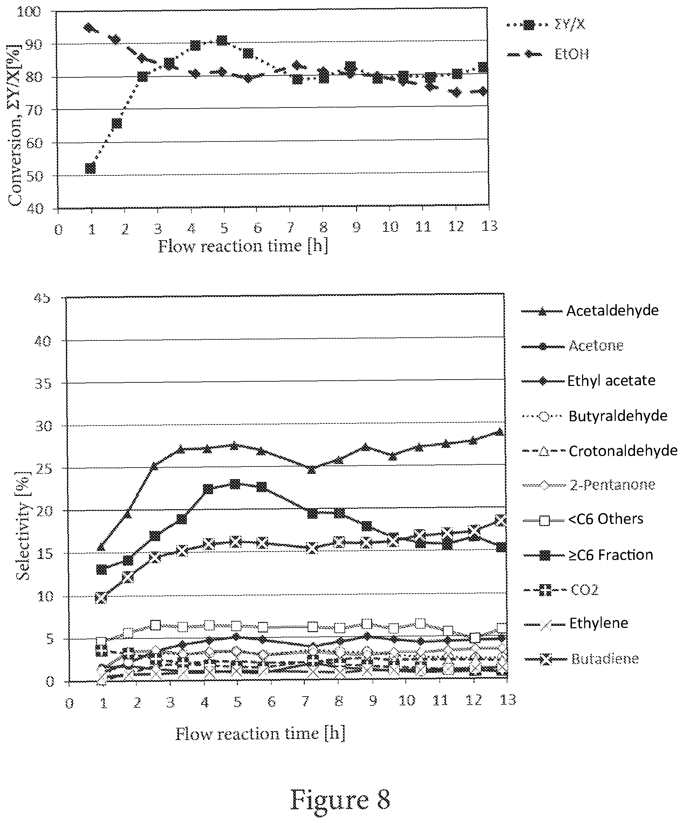

The catalyst employed, Cu/t-ZrO$_2$ with 5% by weight of Cu, was synthesised as in example 1. The catalytic test was carried out as shown in example 2, modifying the overall volumetric flow and the amount of loaded catalyst so as to obtain tau = 3 s. The results are shown in Figure 8.

**Example 5:** *catalytic test under the conditions: Cu/t-ZrO$_2$ with 1% by weight of Cu, 300°C, $\tau$=2 s*

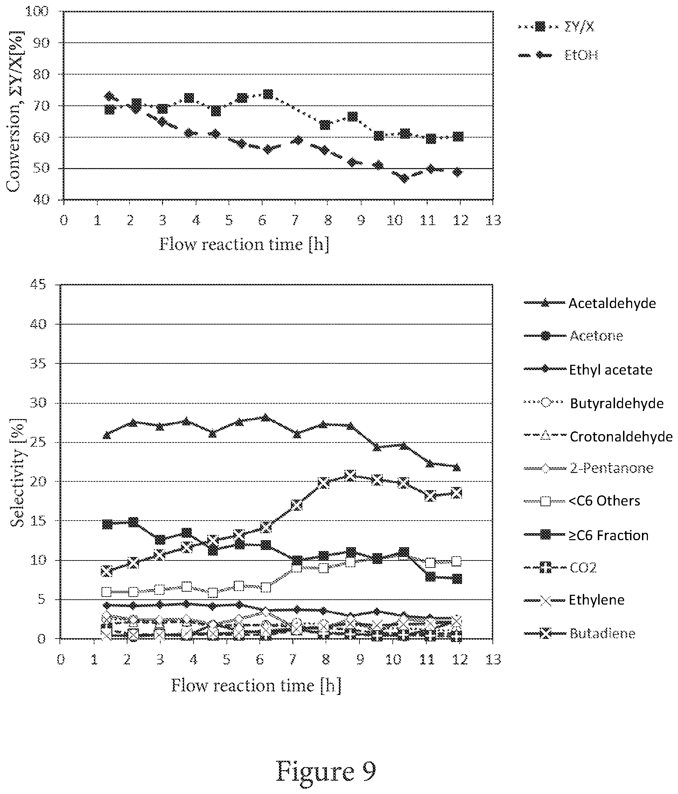

The catalyst employed, Cu/ZrO$_2$ with 1% by weight of Cu, was synthesised as in example 1, modifying the amount of copper precursor used in the IWI in order to obtain a total copper load of 1% by weight of the final material. The catalytic test was carried out as shown in example 3 (tau = 2 s). The results are shown in Figure 9.

*__Example 6:__ catalytic test under the conditions: Cu/t-ZrO$_2$ with 10% by weight of Cu, 300°C, $\tau$=2 s*

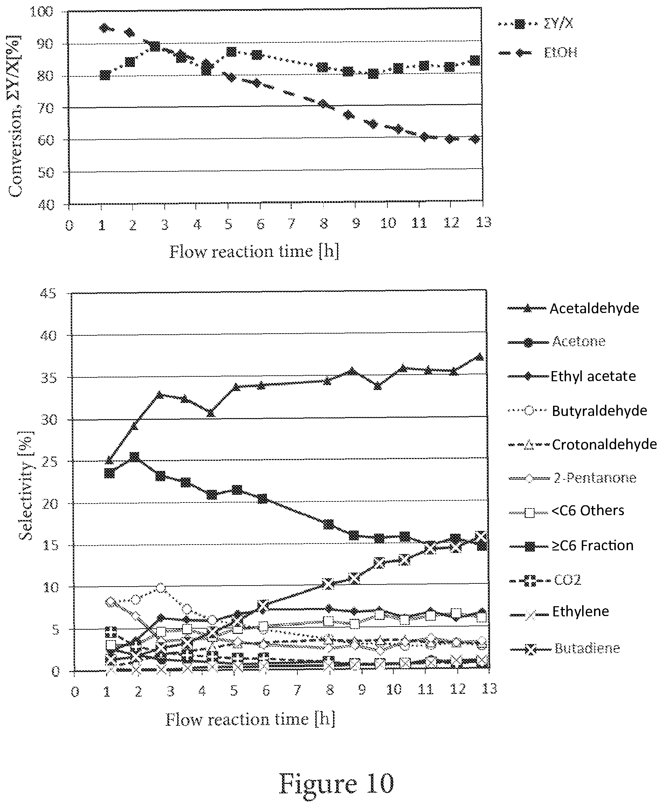

The catalyst employed, Cu/ZrO$_2$ with 10% by weight of Cu, was synthesised as in example 1, modifying the amount of copper precursor used in the IWI in order to obtain a total copper load of 10 wt% of the final material. The catalytic test was carried out as shown in example 3 (tau = 2 s). The results are shown in Figure 10.

*__Example 7:__ Catalytic test under the conditions: CuO/t-ZrO$_2$ with 5% by weight of Cu, 300°C, $\tau$=2 s*

The catalyst employed, CuO/t-ZrO$_2$ with 5% by weight of Cu, was synthesised as in example 1, with the exclusion of the last reduction step which was not carried out. Therefore, a suitable amount of material is loaded into the plant operating continuously in the vapour phase as described in example 2 and used directly, keeping the active phase in the form of cupric oxide. The catalytic test was carried out as shown in example 3 (tau = 2 s). The ethanol conversion averaged over the first 6 h of reaction is 62% and the distribution of the products is shown in Table 3

**Table 3:** *Results of the catalytic test under the conditions: CuO/t-ZrO$_2$ with 5% by weight of Cu, 10 mol% EtOH in He, contact time 2 s, 300°C.*

| Compound | Selectivity averaged over 6 h |
|---|---|
| Acetaldehyde | 46% |
| Ethyl acetate | 5% |
| Butyraldehyde | 4% |
| Crotonaldehyde | 5% |
| 2-pentanone | 5% |
| <C$_6$ others | 3% |
| ≥C$_6$ fraction | 18% |

*__Example 8__: Synthesis of Cu/m-ZrO$_2$ with 5% by weight of Cu.*

The ZrO$_2$ support was obtained by hydrothermal synthesis in an autoclave at 140°C for 20 h at autogenous pressure starting from an aqueous solution of ZrO(NO$_3$)$_2$ and urea. The product is washed several times with ethanol, then it is dried and calcined at 450°C for 3 hours, at a ramp rate of 5°C/min. In this way it is obtained zirconia in the monoclinic crystalline phase. The Cu was deposited on the support with the incipient wetness impregnation (IWI) method, by dissolving the amount of precursor (Cu(NO$_3$)$_2$) necessary to obtain a copper load of 5% by weight of the final catalyst in a volume of water equal to the volume of the pores of the support. This solution is added drop by drop and evenly distributed on the support until it reaches the slurry point (filling of the pores). After which the sample is dried in a furnace and calcined at 450°C for 5 hours, at a ramp rate of 2°C/min.

The powder thus obtained is subjected to a pelletising process so as to obtain particles of a defined size (20-40 mesh). A suitable amount of material is loaded into the plant operating continuously in the vapour phase as described in example 2 and subjected to a reduction process in a hydrogen flow at 350°C for 3 hours so as to obtain the desired catalyst.

*__Example 9:__ catalytic test under the conditions: Cu/m-ZrO$_2$ with 5% by weight of Cu, 300°C, $\tau$=2 s*

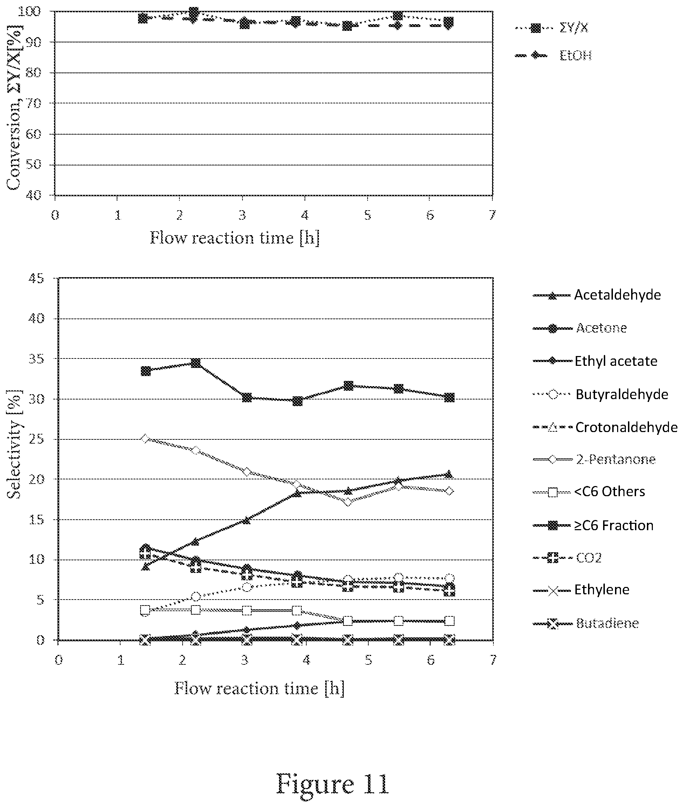

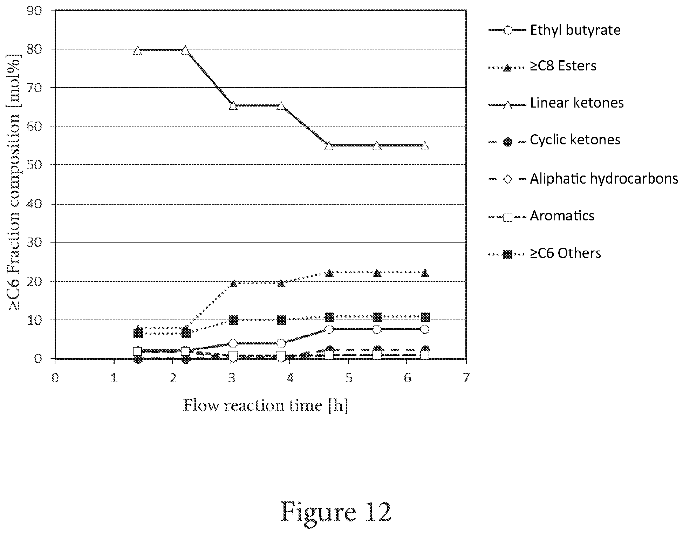

The catalyst employed, Cu/m-ZrO$_2$ with 5% by weight of Cu, was synthesised as in example 8. The catalytic test was carried out as shown in example 3 (tau = 2 s) and the results are shown in Figure 11. The ≥ C$_6$ fraction composition is shown in Figure 12.

Under the same reaction conditions shown above, the use of the Cu/m-ZrO$_2$ catalyst with 5% by weight of Cu (5% by weight of metallic copper supported on monoclinic zirconia) makes it possible to obtain a different distribution of products and a greater stability of the catalyst. During the first 6 hours of reaction the conversion stands at 96% with the following selectivity in the products.

Tables 4 and 5 summarise the results shown in Figures 11 and 12.

**Table 4:** *reaction mixture composition exiting the plant. Reaction conditions: 300°C, contact time: 2 s, 10 mol% EtOH in He, Cu/m-ZrO$_2$ with 5% by weight of Cu. Ethanol conversion: 98% after 2 h, 97% averaged over the first 6 h.*

| Compound | Selectivity after 2 h | Selectivity averaged over 6 h |
|---|---|---|
| Acetaldehyde | 13% | 17% |
| Acetone | 10% | 9% |
| Ethyl acetate | 1% | 2% |
| Butyraldehyde | 5% | 7% |
| 2-pentanone | 23% | 21% |
| CO$_2$ | 9% | 8% |
| <C$_6$ others | 4% | 4% |
| ≥C$_6$ fraction | 35% | 32% |

The "≥C6 fraction" is further divided into the following classes of compounds (Table 5). The composition is expressed as a molar fraction in percentage of the total ≥C$_6$ fraction (T$_{eb}$>120°C) obtained by removing the <C$_6$ fraction with T$_{eb}$<120°C. The ≥C6 others fraction consists mainly of higher alcohols and aldehydes, together with other minority compounds.

**Table 5:** *details about the "≥C6 fraction" composition. Reaction conditions: 300°C, contact time: 2 s, 10 mol% EtOH in He, Cu/m-ZrO$_2$ with 5% by weight of Cu.*

| Compound | Molar fraction % after 2 h | Molar fraction % averaged over 6 h |
|---|---|---|
| Ethyl butyrate | 2% | 5% |
| ≥C$_8$ esters | 8% | 15% |
| Linear ketones | 80% | 67% |
| Cyclic ketones | / | 1% |
| Aliphatic hydrocarbons | 1% | 1% |
| Aromatics | 2% | 2% |
| ≥C$_6$ others | 7% | 9% |

**_Example 10_**: synthesis of Cu/La-Zr-O with 5% by weight of Cu.

The La-Zr-O support was synthesised by precipitation in a basic environment at 25°C starting from an aqueous solution of ZrO(NO$_3$)$_2$*2H$_2$O and La(NO$_3$)$_3$*6H$_2$O such that the atomic ratio La:Zr in the final material was 0.19 and the total cation concentration was 0.3 mol/l. The suspension was subjected to a digestion of 72 h at 100°C and at controlled pH 10-12 by continuously adding a concentrated ammonia aqueous solution (28% w/w). The sample was then filtered, dried and calcined at 450°C for 12 h at a ramp rate of 5°C/min. The Cu was deposited on the support using the incipient wetness impregnation (IWI) method, by dissolving the amount of precursor (Cu(NO$_3$)$_2$) necessary to obtain a copper load of 5 wt% on the final catalyst in a water volume equal to the pore volume of the support. This solution is added drop by drop and evenly distributed on the support until it reaches the slurry point (filling of the pores). After which the sample is dried in a furnace and calcined at 450°C for 5 h, at a ramp rate of 2°C/min.

The powder thus obtained is subjected to a pelletising process so as to obtain particles of a defined size (20-40 mesh). A suitable amount of material is loaded into the plant operating continuously in the vapour phase as described in example 2 and subjected to a reduction process in a hydrogen flow at 350°C for 3 hours so as to obtain the desired catalyst.

**_Example 11_**: catalytic test with Cu/La-Zr-O with 5% by weight of Cu, 300°C, $\tau$=2 s

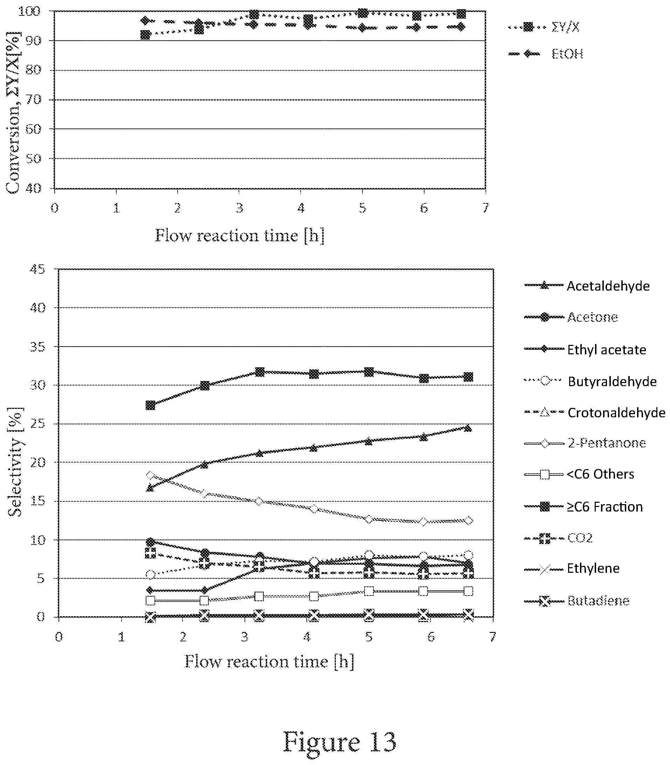

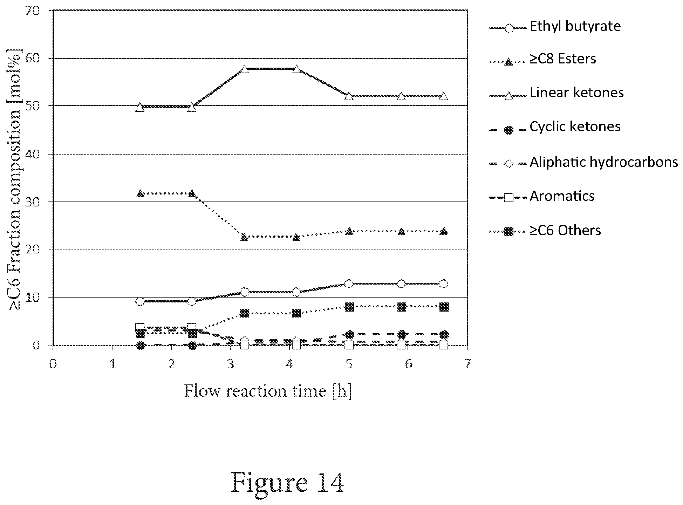

The catalyst was synthesised as in example 9. The catalytic test was carried out as shown in example 3 (tau = 2 s) and the results are shown in Figure 13. The ≥C6 fraction composition is shown in Figure 14.

***Example 12:*** *catalytic test with Cu/t-ZrO$_2$ with 5% by weight of Cu, 300°C, $\tau$=2 s, EtOH in He:H$_2$ flow in a 1:1 ratio*

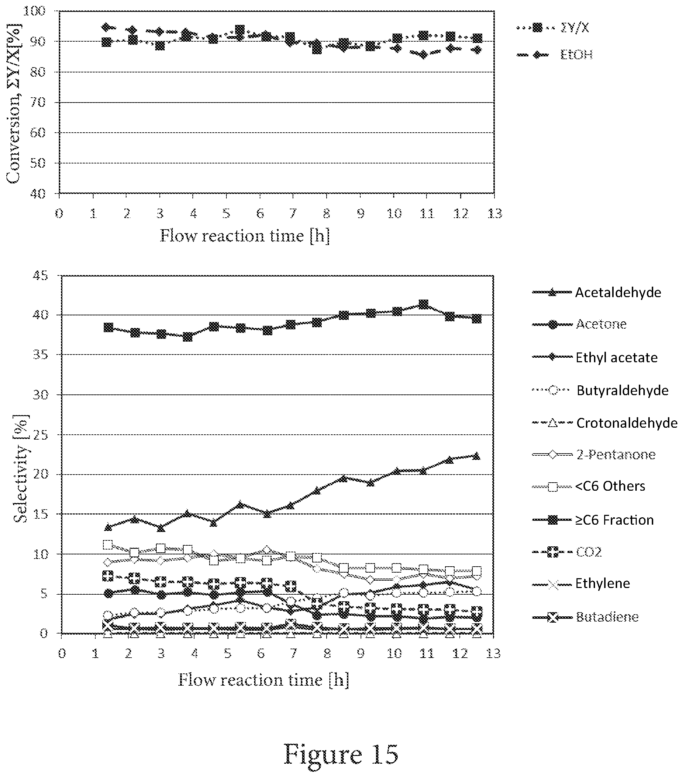

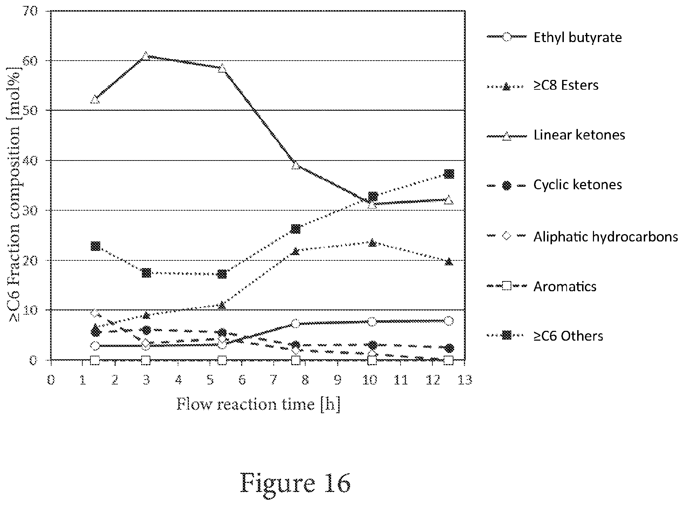

The catalyst employed, Cu/t-ZrO$_2$ with 5% by weight of Cu, was synthesised as in example 1. The catalytic test was carried out as shown in example 3 (tau = 2 s), but the carrier gas was modified from 100% He (as per example 3) to a mixture of He and H$_2$ in a 1:1 ratio. The molar percent of EtOH was maintained at 10 mol% of the total fed moles. The results of the catalytic test are shown in Figure 15. The ≥C6 fraction composition is shown in Figure 16.

***Example 13:*** *catalytic test with Cu/t-ZrO$_2$ with 5% by weight of Cu, 300°C, $\tau$=2 s, 1-butanol (10 mol%) in He flow.*

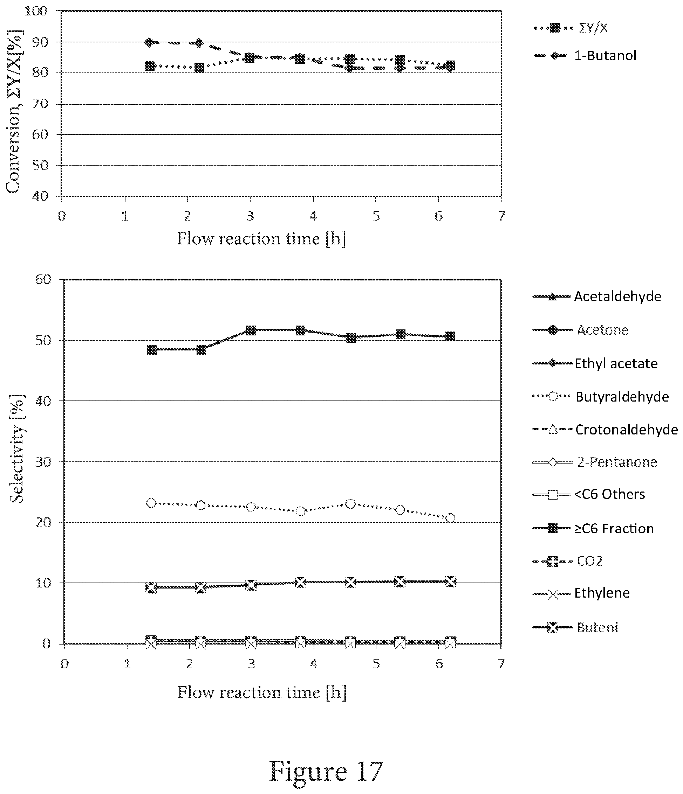

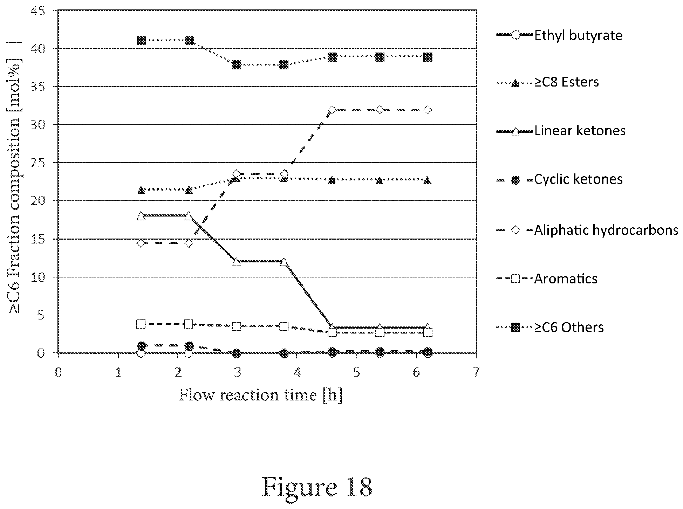

The catalyst employed, Cu/t-ZrO$_2$ with 5% by weight of Cu, was synthesised as in example 1. The catalytic test was carried out as shown in example 3, modifying the reactant alcohol, i.e. feeding 1- butanol instead of ethanol. The results are shown in Figure 17. The ≥ C6 fraction composition is shown in Figure 18.

The present invention has been described for illustrative, but non-limiting purposes, according to its preferred embodiments, but it is to be understood that variations and/or modifications can be made by those skilled in the art without thereby departing from its scope of protection, as defined by the attached claims.

**Bibliography**

- Patent US 10221119 B2, Karthikeyan Kallupalayam Ramasamy, Michel J. Gray, Carlos A. Alvarez - Vasco, Mond F. Guo, Senthil Subramaniam, Conversion of ethanol to C5+ ketones in single catalyst bed, 2019
- Patent US 10745330 B2, Karthikeyan K. Ramasamy, Mond F. Guo, Michel J. Gray, Senthil Subramaniam, Method of converting ethanol to higher alcohols, 2020
- Patent Application Publication US 20160194257 A1, Michael A. Lilga, Richard T. Hallen, Karl O. Albrecht, Alan R. Cooper, John G. Frye, Karthi Ramasamy, Systems and processes for conversion of ethylene feedstocks to hydrocarbon fuels, 2016
- Patent US 10815163 B2, Charles E. Wyman, John R. Hannon, Systems for reducing resource consumption in production of alcohol fuel by conversion to hydrocarbon fuels, 2020
- Patent Application Publication US 20160362612 A1, Charles E. Wyman, John R. Hannon, Systems and methods for reducing energy consumption in production of ethanol fuel by conversion to hydrocarbon fuels, 2016
- International Publication Number WO 2017173165 A1, Charles E. Wyman, John R. Hannon, Systems and methods for improving yields of hydrocarbon fuels from alcohols, 2017
- International Publication Number WO 2016201297 A1, Charles E. Wyman, John R. Hannon, Systems and methods for reducing resource consumption in production of alcohol fuel by conversion to hydrocarbon fuels, 2016
- S. Subramaniam, M. F. Guo, T. Bathena, M. Gray, X. Zhang, A. Martinez, L. Kovarik, K. A. Goulas and K. K. Ramasamy, Direct Catalytic Conversion of Ethanol to C5+ Ketones: Role of PdZn Alloy on Catalytic Activity and Stability, Angewandte Chemie International Edition, 2020, 59, 14550-14557.
- G. Pomalaza et al. Ethanol-to-butadiene: the reaction and its catalysts. Catal. Sci. Technol., 2020, 10, 4860
- N. Scotti, F. Zaccheria, C. Evangelisti, R. Psaro and N. Ravasio, Dehydrogenative coupling promoted by copper catalysts: a way to optimise and upgrade bioalcohols, Catal. Sci. Technol., 2017, 7, 1386-1393.
- N. Scotti, N. Ravasio, F. Zaccheria, A. Irimescu and S. S. Merola, Green pathway to a new fuel extender: continuous flow catalytic synthesis of butanol/butyl butyrate mixtures, RSC Adv., 2020, 10, 3130-3136.
- N. Scotti, F. Bossola, F. Zaccheria and N. Ravasio, Copper-Zirconia Catalysts: Powerful Multifunctional Catalytic Tools to Approach Sustainable Processes, Catalysts, 2020, 10, 168.
- F. Zaccheria, N. Scotti, and N. Ravasio, The role of Copper in the upgrading of Bio alcohols, ChemCatChem 10.1002/cctc.201701844
- Y. Zhu, J. Zheng, J. Ye, Y. Cui, K. Koh, L. Kovarik, D. M. Camaioni, J. L. Fulton, D. G. Truhlar, M. Neurock, C. J. Cramer, O. Y. Gutiérrez and J. A. Lercher, Copper-zirconia interfaces in UiO-66 enable selective catalytic hydrogenation of CO2 to methanol, Nature Communications, 2020, 11, 5849.
- C. Mateos-Pedrero, C. Azenha, P. T. D.a., J. M. Sousa and A. Mendes, The influence of the support composition on the physicochemical and catalytic properties of Cu catalysts supported on Zirconia-Alumina for methanol steam reforming, Applied Catalysis B: Environmental, 2020, 277, 119243.
- M. Sliwa and K. Samson, Steam reforming of ethanol over copper-zirconia based catalysts doped with Mn, Ni, Ga, International Journal of Hydrogen Energy, DOI:10.1016/j.ijhydene.2020.09.222.
- V. Shahed Gharahshiran, M. Yousefpour and V. Amini, A comparative study of zirconia and yttria promoted mesoporous carbonnickel- cobalt catalysts in steam reforming of ethanol for hydrogen production, Molecular Catalysis, 2020, 484, 110767.

- F. Bossola, N. Scotti, F. Somodi, M. Coduri, C. Evangelisti and V. Dal Santo, Electron-poor copper nanoparticles over amorphous zirconia-silica as all-in-one catalytic sites for the methanol steam reforming Applied Catalysis B: Environmental, 2019, 258, 118016.

- A. Bialas, T. Kondratowicz, M. Drozdek and P. Kuśtrowski, Catalytic combustion of toluene over copper oxide deposited on twotypes of yttria-stabilized zirconia, Catalysis Today, 2015, 257, 144-149.

- T. Tsoncheva, I. Genova, M. Dimitrov, E. Sarcadi-Priboczki, A. M. Venezia, D. Kovacheva, N. Scotti and V. dal Santo, Nanostructured copper-zirconia composites as catalysts for methanol decomposition Applied Catalysis B: Environmental, 2015, 165, 599-610.

- K. A. Ali, A. Z. Abdullah and A. R. Mohamed, Recent development in catalytic technologies for methanol synthesis from renewable sources: A critical review, Renewable and Sustainable Energy Reviews, 2015, 44, 508-518.

- K. Li and J. G. Chen, $CO_2$ Hydrogenation to Methanol over $ZrO_2$-Containing Catalysts: Insights into $ZrO_2$ Induced Synergy, ACS Catal., 2019, 9, 7840-7861.

- P. Liu, Y. Yang and M. G. White, Theoretical perspective of alcohol decomposition and synthesis from $CO_2$ hydrogenation, Surface Science Reports, 2013, 68, 233-272.

- K. Inui, T. Kurabayashi and S. Sato, Direct synthesis of ethyl acetate from ethanol carried out under pressure, Journal of Catalysis, 2002, 212, 207-215.

- A. G. Sato, D. P. Volanti, D. M. Meira, S. Damyanova, E. Longo and J. M. C. Bueno, Effect of the $ZrO_2$ phase on the structure and behavior of supported Cu catalysts for ethanol conversion, Journal of Catalysis, 2013, 307, 1-17.

- Gui-Sheng Wu, Dong-Sen Mao, Guan-Zhong Lu, Yong Cao, Kang-Nian Fan, The Role of the Promoters in Cu Based Catalysts for Methanol Steam Reforming, Catal Lett, 2009, 130, 177-184

- K. Inui, T. Kurabayashi and S. Sato, Direct synthesis of ethyl acetate from ethanol over Cu-Zn-Zr-Al-O catalyst, Applied Catalysis A: General, 2002, 237, 53-61.

- Insoo Ro, Yifei Liu, Madelyn R. Ball, David H. K. Jackson, Joseph Paul Chada, Canan Sener, Thomas F. Kuech, Rostam J. Madon, George W. Huber, and James A. Dumesic, Role of the Cu-$ZrO_2$ Interfacial Sites for Conversion of Ethanol to Ethyl Acetate and Synthesis of Methanol from $CO_2$ and $H_2$, ACS Catal. 2016, 6, 7040-7050

- H. Miura, K. Nakahara, T. Kitajima and T. Shishido, Concerted Functions of Surface Acid-Base Pairs and Supported Copper Catalysts for Dehydrogenative Synthesis of Esters from Primary Alcohols, ACS Omega, 2017, 2, 6167-6173.

- A. G. Sato, D. P. Volanti, I. C. de Freitas, E. Longo and J. M. C. Bueno, Site-selective ethanol conversion over supported copper catalysts, Catalysis Communications, 2012, 26, 122-126.

- K. Samson, M. Sliwa, R. P. Socha, K. Góra-Marek, D. Mucha, D. Rutkowska-Zbik, J-F. Paul, M. Ruggiero-Mikolajczyk, R. Grabowski, and J. Sloczyński, Influence of $ZrO_2$ Structure and Copper Electronic State on Activity of Cu/$ZrO_2$ Catalysts in Methanol Synthesis from $CO_2$, ACS Catal. 2014, 4, 3730-3741

- G. Prieto, J. Zečević, H. Friedrich, K. P. de Jong and P. E. de Jongh, Towards stable catalysts by controlling collective properties of supported metal nanoparticles, Nat Mater, 2013, 12, 34-39.

- J. Zhou, Y. Zhang, G. Wu, D. Mao and G. Lu, Influence of the component interaction over Cu/$ZrO_2$ catalysts induced with fractionated precipitation method on the catalytic performance for methanol steam reforming, RSC Adv., 2016, 6, 30176-30183.

- C.Z. Yao, L.C. Wang, Y.M. Liu, G.S. Wu, Y. Cao, W.L. Dai, H.Y. He and K.N. Fan, Effect of preparation method on the hydrogen production from methanol steam reforming over binary Cu/$ZrO_2$ catalysts, Applied Catalysis A: General, 2006, 297, 151-158.

- R. Raudaskoski, E. Turpeinen, R. Lenkkeri, E. Pongra' cz, R.L. Keiski, Catalytic activation of $CO_2$: Use of secondary $CO_2$ for the production of synthesis gas and for methanol synthesis over copper-based zirconia-containing catalysts, Catalysis Today 144 (2009) 318-323

## Claims

1. Process for the production of a mixture comprising compounds having a number of carbon atoms $\geq 6$, usable as jet fuel or as a jet fuel precursor, starting from one or more alcohols having from 2 to 4 carbon atoms, preferably ethanol, wherein said one or more alcohols are introduced into a continuously operated fixed bed catalyst reactor, in an amount from 1 to 40 mol%, in the vapour phase, at a temperature ranging from 200°C to 450°C and wherein said one or more alcohols are placed in contact with said catalyst for a contact time $\tau$, wherein $\tau$ = catalyst volume (mL)/total volumetric flow (mL/s), said contact time $\tau$ ranging from 1.5 to 5 seconds,
said catalyst comprising metallic copper on a zirconia-based support, said metallic copper being in a percentage by weight ranging from 0.5% to 25% of the total weight of the catalyst.

2. Process according to the preceding claim, wherein said contact time $\tau$ ranges from 1.5 to 3 seconds.

3. Process according to any one of claims 1-2, wherein said contact time $\tau$ ranges from 1.85 to 2.53 seconds.

4. Process according to any one of claims 1-3, wherein said contact time $\tau$ is 2 seconds.

5. Process according to any one of the preceding claims, wherein said process is carried out at a pressure ranging from atmospheric pressure to 10 bar.

6. Process according to the preceding claim, wherein said process is carried out at a pressure ranging from atmospheric pressure to 5 bar.

7. Process according to any one of claims 5-6, wherein said process is carried out at a pressure ranging from atmospheric pressure to 2 bar.

8. Process according to any one of claims 5-7, wherein said process is carried out at atmospheric pressure.

9. Process according to the preceding claim, wherein said one or more alcohols are introduced into said reactor in an amount from 1 to 25 mol%.

10. Process according to the preceding claim, wherein said one or more alcohols are introduced into said reactor in an amount of 10 mol%.

11. Process according to any one of the preceding claims, wherein said temperature ranges from 250 to 340°C.

12. Process according to the preceding claim, wherein said temperature is 300°C.

13. Process according to any one of the preceding claims, wherein said metallic copper is in a percentage by weight from 1% to 10%.

14. Process according to the preceding claim, wherein said metallic copper is in a percentage by weight of 5%.

15. Process according to any one of the preceding claims, wherein said one or more alcohols are introduced into said reactor in an inert gas flow, in particular consisting of helium, nitrogen, argon and/or other gaseous component inert to the reaction conditions, in particular water vapour or $CO_2$.

16. Process according to any one of the preceding claims, wherein said zirconia is tetragonal zirconia or monoclinic zirconia.

17. Process according to any one of the preceding claims, wherein said zirconia-based support comprises a lanthanide included in the structure of said zirconia.

18. Process according to the preceding claim, wherein the Zr:lanthanide atomic ratio ranges from 50:1 to 1:1, preferably from 20:1 to 2:1, more preferably being 5:1.

19. Process according to any one of the preceding claims, wherein said catalyst is selected from a catalyst comprising metallic copper on a tetragonal zirconia-based support wherein the metallic copper is present in an amount of 5% by weight of the total weight of the catalyst; a catalyst comprising metallic copper on a tetragonal zirconia-based support wherein metallic copper is present in an amount of 1% by weight of the total weight of the catalyst; a catalyst comprising metallic copper on a tetragonal zirconia-based support wherein the metallic copper is present in an amount of 10% by weight of the total weight of the catalyst; a catalyst comprising metallic copper on a monoclinic zirconia-based support wherein the metallic copper is present in an amount of 5% by weight of the total weight of the catalyst; a catalyst comprising metallic copper on a zirconia-based support comprising lanthanum included in the structure of said zirconia wherein the metallic copper is present in an amount of 5% by weight of the total weight of the catalyst.

20. Process according to any one of the preceding claims, wherein hydrogen is fed together with said one or more alcohols or, when said one or more alcohols are introduced into the reactor in an inert gas flow, together with said inert gas flow.

21. Process according to the preceding claim, wherein the inert gas:$H_2$ ratio ranges from 1:10 to 10:1.

22. Process according to any one of the preceding claims, wherein said catalyst is in the form of powder or pellet.

23. Process according to any one of the preceding claims, wherein said mixture comprises esters, in particular linear and branched esters, alcohols, in particular branched and linear alcohols, ketones, in particular branched, linear and cyclic ketones, aldehydes, aliphatic hydrocarbons, such as for example alkanes and olefins, and aromatic compounds, in particular phenolic compounds.

24. Process according to any one of the preceding claims, said process further comprising a hydrogenation step of the aldehydes and unsaturated compounds and, optionally, a dehydration step.

**Patentansprüche**

1. Verfahren zur Herstellung eines Gemisches, das Verbindungen mit einer Anzahl von Kohlenstoffatomen ≥6 enthält und als Düsentreibstoff oder als Düsentreibstoffvorläufer verwendbar ist, ausgehend von einem oder mehreren Alkoholen mit 2 bis 4 Kohlenstoffatomen, vorzugsweise Ethanol, wobei der eine oder die mehreren Alkohole in einen kontinuierlich betriebenen Festbettkatalysatorreaktor eingeführt werden, in einer Menge von 1 bis 40 Mol-% in der Dampfphase bei einer Temperatur im Bereich von 200°C bis 450°C eingebracht werden und wobei der eine oder die mehreren Alkohole mit dem Katalysator für eine Kontaktzeit $_T$ in Kontakt gebracht werden, wobei $_T$ = Katalysatorvolumen (mL)/Gesamtvolumenstrom (mL/s), wobei die Kontaktzeit $_T$ im Bereich von 1.5 bis 5 Sekunden liegt; wobei der Katalysator metallisches Kupfer auf einem Träger auf Zirkoniumdioxidbasis umfasst, wobei das metallische Kupfer in einem Gewichtsprozentsatz von 0.5 bis 25% des Gesamtgewichts des Katalysators vorliegt.

2. Verfahren nach dem vorhergehenden Anspruch, wobei die Kontaktzeit $_T$ zwischen 1.5 und 3 Sekunden liegt.

3. Verfahren nach einem der Ansprüche 1-2, wobei die Kontaktzeit $_T$ zwischen 1.85 und 2.53 Sekunden liegt.

4. Verfahren nach einem der Ansprüche 1-3, wobei die Kontaktzeit $_T$ 2 Sekunden beträgt.

5. Verfahren nach einem der vorhergehenden Ansprüche, wobei das Verfahren bei einem Druck im Bereich von Atmosphärendruck bis 10 bar durchgeführt wird.

6. Verfahren nach dem vorhergehenden Anspruch, wobei das Verfahren bei einem Druck im Bereich von Atmosphärendruck bis 5 bar durchgeführt wird.

7. Verfahren nach einem der Ansprüche 5-6, wobei das Verfahren bei einem Druck im Bereich von Atmosphärendruck bis 2 bar durchgeführt wird.

8. Verfahren nach einem der Ansprüche 5-7, wobei das Verfahren bei Atmosphärendruck durchgeführt wird.

9. Verfahren nach dem vorhergehenden Anspruch, wobei der eine oder die mehreren Alkohole in einer Menge von 1 bis 25 Mol-% in den Reaktor eingeführt werden.

10. Verfahren nach dem vorhergehenden Anspruch, wobei der eine oder die mehreren Alkohole in einer Menge von 10 Mol-% in den Reaktor eingeführt werden.

11. Verfahren nach einem der vorhergehenden Ansprüche, wobei die Temperatur im Bereich von 250 bis 340°C liegt.

12. Verfahren nach dem vorhergehenden Anspruch, wobei die Temperatur 300°C beträgt.

13. Verfahren nach einem der vorhergehenden Ansprüche, wobei das metallische Kupfer in einem Gewichtsprozentanteil von 1% bis 10% vorliegt.

14. Verfahren nach dem vorhergehenden Anspruch, wobei das metallische Kupfer in einem Gewichtsprozentanteil von 5% vorliegt.

15. Verfahren nach einem der vorhergehenden Ansprüche, wobei der eine oder die mehreren Alkohole in einem Inertgasstrom in den Reaktor eingeführt werden, der insbesondere aus Helium, Stickstoff, Argon und/oder einer anderen gasförmigen Komponente besteht, die gegenüber den Reaktionsbedingungen inert ist, insbesondere Wasserdampf oder $CO_2$.

16. Verfahren nach einem der vorhergehenden Ansprüche, wobei das Zirkoniumdioxid tetragonales Zirkoniumdioxid oder monoklines Zirkoniumdioxid ist.

17. Verfahren nach einem der vorhergehenden Ansprüche, wobei der Träger auf Zirkoniumdioxidbasis ein Lanthanid umfasst, das in der Struktur des Zirkoniumdioxids enthalten ist.

18. Verfahren nach dem vorhergehenden Anspruch, wobei das Zr:Lanthanid-Atomverhältnis im Bereich von 50:1 bis 1:1, vorzugsweise von 20:1 bis 2:1, besonders bevorzugt bei 5:1 liegt.

19. Verfahren nach einem der vorstehenden Ansprüche, wobei der Katalysator ausgewählt ist aus einem Katalysator, der metallisches Kupfer auf einem tetragonalen Träger auf Zirkoniumoxidbasis umfasst, wobei das metallische Kupfer in einer Menge von 5 Gew.-% des Gesamtgewichts des Katalysators vorliegt; einem Katalysator, der metallisches Kupfer auf einem tetragonalen Träger auf Zirkoniumoxidbasis umfasst, wobei das metallische Kupfer in einer Menge von 1 Gew.-% des Gesamtgewichts des Katalysators vorliegt; einem Katalysator, der metallisches Kupfer auf einem tetragonalen Träger auf Zirkoniumoxidbasis umfasst, wobei das metallische Kupfer in einer Menge von 10 Gew.-% des Gesamtgewichts des Katalysators vorliegt; einen Katalysator, der metallisches Kupfer auf einem monoklinen Träger auf Zirkoniumoxidbasis umfasst, wobei das metallische Kupfer in einer Menge von 5 Gew.-% des Gesamtgewichts des Katalysators vorliegt; einen Katalysator, der metallisches Kupfer auf einem Träger auf Zirkoniumoxidbasis umfasst, der Lanthan enthält, das in die Struktur des Zirkoniumoxids eingebaut ist, wobei das metallische Kupfer in einer Menge von 5 Gew.-% des Gesamtgewichts des Katalysators vorliegt.

20. Verfahren nach einem der vorhergehenden Ansprüche, wobei Wasserstoff zusammen mit dem einen oder den mehreren Alkoholen oder, wenn der eine oder die mehreren Alkohole in einem Inertgasstrom in den Reaktor eingeführt werden, zusammen mit dem Inertgasstrom zugeführt wird.

21. Verfahren nach dem vorhergehenden Anspruch, wobei das Verhältnis von Inertgas:$H_2$ im Bereich von 1:10 bis 10:1 liegt.

22. Verfahren nach einem der vorhergehenden Ansprüche, wobei der Katalysator in Form von Pulver oder Pellets vorliegt.

23. Verfahren nach einem der vorhergehenden Ansprüche, **dadurch gekennzeichnet, dass** das Gemisch Ester, insbesondere lineare und verzweigte Ester, Alkohole, insbesondere verzweigte und lineare Alkohole, Ketone, insbesondere verzweigte, lineare und cyclische Ketone, Aldehyde, aliphatische Kohlenwasserstoffe, wie z.B. Alkane und Olefine, und aromatische Verbindungen, insbesondere Phenolverbindungen, enthält.

24. Verfahren nach einem der vorhergehenden Ansprüche, wobei das Verfahren ferner einen Hydrierungsschritt der Aldehyde und ungesättigten Verbindungen und gegebenenfalls einen Dehydrierungsschritt umfasst.

**Revendications**

1. Procédé de production d'un mélange comprenant des composés ayant un nombre d'atomes de carbone ≥6, utilisable comme carburéacteur ou comme précurseur de carburéacteur, à partir d'un ou plusieurs alcools ayant de 2 à 4 atomes de carbone, de préférence de l'éthanol, dans lequel ledit ou lesdits alcools sont introduits dans un réacteur catalytique à lit fixe fonctionnant en continu, dans une proportion de 1 à 40% en phase vapeur, à une température comprise entre 200°C et 450°C, et dans lequel un ou plusieurs alcools sont mis en contact avec ledit catalyseur pendant un temps de contact $_T$, dans lequel $_T$ = volume du catalyseur (mL)/débit volumétrique total (mL/s), ledit temps de contact $_T$ étant compris entre 1.5 à 5 secondes; ledit catalyseur comprend du cuivre métallique sur un support à base de zircone, ledit cuivre métallique représentant un pourcentage en poids allant de 0.5% à 25% du poids total du catalyseur.

2. Procédé selon la revendication précédente, dans lequel le temps de contact $_T$ est compris entre 1.5 et 3 secondes.

3. Procédé selon l'une des revendications 1-2, dans lequel le temps de contact $_T$ est compris entre 1.85 et 2.53 secondes.

4. Procédé selon l'une des revendications 1-3, dans lequel le temps de contact $_T$ est de 2 secondes.

5. Procédé selon l'une quelconque des revendications précédentes, dans lequel ledit procédé est mis en œuvre à une pression allant de la pression atmosphérique à 10 bars.

6. Procédé selon la revendication précédente, dans lequel ledit procédé est réalisé à une pression allant de la pression atmosphérique à 5 bars.

7. Procédé selon l'une des revendications 5-6, dans lequel ledit procédé est réalisé à une pression comprise entre la pression atmosphérique et 2 bars.

8. Procédé selon l'une des revendications 5-7, dans lequel ledit procédé est effectué à la pression atmosphérique.

9. Procédé selon la revendication précédente, dans lequel un ou plusieurs alcools sont introduits dans le réacteur dans une proportion de 1 à 25% en moles.

10. Procédé selon la revendication précédente, dans lequel un ou plusieurs alcools sont introduits dans le réacteur à raison de 10% en moles.

11. Procédé selon l'une des revendications précédentes, dans lequel la température est comprise entre 250 et 340°C.

12. Procédé selon la revendication précédente, dans lequel la température est de 300°C.

13. Procédé selon l'une quelconque des revendications précédentes, dans lequel ledit cuivre métallique représente un pourcentage en poids de 1% à 10%.

14. Procédé selon la revendication précédente, dans lequel ledit cuivre métallique représente un pourcentage en poids de 5%.

15. Procédé selon l'une quelconque des revendications précédentes, dans lequel ledit ou lesdits alcools sont introduits dans ledit réacteur dans un flux de gaz inerte, notamment constitué d'hélium, d'azote, d'argon et/ou d'un autre composant gazeux inerte par rapport aux conditions de réaction, notamment de la vapeur d'eau ou du $CO_2$.

16. Procédé selon l'une quelconque des revendications précédentes, dans lequel ladite zircone est une zircone tétragonale ou une zircone monoclinique.

17. Procédé selon l'une quelconque des revendications précédentes, dans lequel ledit support à base de zircone comprend un lanthanide inclus dans la structure de ladite zircone.

18. Procédé selon la revendication précédente, dans lequel le rapport atomique Zr:lanthanide est compris entre 50:1 et 1:1, de préférence entre 20:1 et 2:1, plus préférentiellement 5:1.

19. Procédé selon l'une quelconque des revendications précédentes, dans lequel ledit catalyseur est choisi parmi un catalyseur comprenant du cuivre métallique sur un support à base de zircone tétragonale, dans lequel le cuivre métallique est présent en une quantité de 5% en poids du poids total du catalyseur; un catalyseur comprenant du cuivre métallique sur un support à base de zircone tétragonale, dans lequel le cuivre métallique est présent en une quantité de 1% en poids du poids total du catalyseur; un catalyseur comprenant du cuivre métallique sur un support à base de zircone tétragonale, dans lequel le cuivre métallique est présent en une quantité de 10% en poids du poids total du catalyseur; un catalyseur comprenant du cuivre métallique sur un support à base de zircone monoclinique, dans lequel le cuivre métallique est présent en une quantité de 5% en poids par rapport au poids total du catalyseur; un catalyseur comprenant du cuivre métallique sur un support à base de zircone comprenant du lanthane inclus dans la structure de ladite zircone, dans lequel le cuivre métallique est présent en une quantité de 5% en poids par rapport au poids total du catalyseur.

20. Procédé selon l'une quelconque des revendications précédentes, dans lequel l'hydrogène est introduit en même temps que ledit ou lesdits alcools ou, lorsque ledit ou lesdits alcools sont introduits dans le réacteur dans un flux de gaz inerte, en même temps que ledit flux de gaz inerte.

21. Procédé selon la revendication précédente, dans lequel le rapport gaz inerte:$H_2$ est compris entre 1:10 et 10:1.

22. Procédé selon l'une quelconque des revendications précédentes, dans lequel ledit catalyseur se présente sous forme de poudre ou de granulés.

23. Procédé selon l'une quelconque des revendications précédentes, dans lequel ledit mélange comprend des esters, en particulier des esters linéaires et ramifiés, des alcools, en particulier des alcools ramifiés et linéaires, des cétones, en particulier des cétones ramifiées, linéaires et cycliques, des aldéhydes, des hydrocarbures aliphatiques, tels que, par exemple, des alcanes et des oléfines, et des composés aromatiques, en particulier des composés phénoliques.

24. Procédé selon l'une quelconque des revendications précédentes, ledit procédé comprenant en outre une étape d'hydrogénation des aldéhydes et des composés insaturés et, éventuellement, une étape de déshydratation.

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

Figure 16

Figure 17

Figure 18

## REFERENCES CITED IN THE DESCRIPTION

### Patent documents cited in the description

- US 10221119 B2
- US 10815163 B2
- US 10745330 B2
- US 20180215692 A
- US 20160194257 A1, Michael A. Lilga, Richard T. Hallen, Karl O. Albrecht, Alan R. Cooper, John G. Frye, Karthi Ramasamy
- US 20160362612 A1, Charles E. Wyman, John R. Hannon
- WO 2017173165 A1, Charles E. Wyman, John R. Hannon
- WO 2016201297 A1, Charles E. Wyman, John R. Hannon

### Non-patent literature cited in the description

- **S. SUBRAMANIAM ; M. F. GUO ; T. BATHENA ; M. GRAY ; X. ZHANG ; A. MARTINEZ ; L. KOVARIK ; K. A. GOULAS ; K. K. RAMASAMY**. *Angewandte Chemie International Edition*, 2020, vol. 59, 14550-14557
- **S. SUBRAMANIAM ; M. F. GUO ; T. BATHENA ; M. GRAY ; X. ZHANG ; A. MARTINEZ ; L. KOVARIK ; K. A. GOULAS ; K. K. RAMASAMY**. Direct Catalytic Conversion of Ethanol to C5+ Ketones: Role of PdZn Alloy on Catalytic Activity and Stability. *Angewandte Chemie International Edition*, 2020, vol. 59, 14550-14557
- **G. POMALAZA et al.** Ethanol-to-butadiene: the reaction and its catalysts. *Catal. Sci. Technol.*, 2020, vol. 10, 4860
- **N. SCOTTI ; F. ZACCHERIA ; C. EVANGELISTI ; R. PSARO ; N. RAVASIO**. Dehydrogenative coupling promoted by copper catalysts: a way to optimise and upgrade bioalcohols. *Catal. Sci. Technol.*, 2017, vol. 7, 1386-1393
- **N. SCOTTI ; N. RAVASIO ; F. ZACCHERIA ; A. IRIMESCU ; S. S. MEROLA**. Green pathway to a new fuel extender: continuous flow catalytic synthesis of butanol/butyl butyrate mixtures. *RSC Adv.*, 2020, vol. 10, 3130-3136
- **N. SCOTTI ; F. BOSSOLA ; F. ZACCHERIA ; N. RAVASIO**. Copper-Zirconia Catalysts: Powerful Multifunctional Catalytic Tools to Approach Sustainable Processes. *Catalysts*, 2020, vol. 10, 168
- **F. ZACCHERIA ; N. SCOTTI ; N. RAVASIO**. The role of Copper in the upgrading of Bio alcohols. *ChemCatChem*
- **Y. ZHU ; J. ZHENG ; J. YE ; Y. CUI ; K. KOH ; L. KOVARIK ; D. M. CAMAIONI ; J. L. FULTON ; D. G. TRUHLAR ; M. NEUROCK**. Copper-zirconia interfaces in UiO-66 enable selective catalytic hydrogenation of CO2 to methanol. *Nature Communications*, 2020, vol. 11, 5849
- **C. MATEOS-PEDRERO ; C. AZENHA ; P. T. D.A. ; J. M. SOUSA ; A. MENDES**. The influence of the support composition on the physicochemical and catalytic properties of Cu catalysts supported on Zirconia-Alumina for methanol steam reforming. *Applied Catalysis B: Environmental*, 2020, vol. 277, 119243
- **M. SLIWA ; K. SAMSON**. Steam reforming of ethanol over copper-zirconia based catalysts doped with Mn, Ni, Ga. *International Journal of Hydrogen Energy*
- **V. SHAHED GHARAHSHIRAN ; M. YOUSEFPOUR ; V. AMINI**. A comparative study of zirconia and yttria promoted mesoporous carbonnickel- cobalt catalysts in steam reforming of ethanol for hydrogen production. *Molecular Catalysis*, 2020, vol. 484, 110767
- **F. BOSSOLA ; N. SCOTTI ; F. SOMODI ; M. CODURI ; C. EVANGELISTI ; V. DAL SANTO**. Electron-poor copper nanoparticles over amorphous zirconia-silica as all-in-one catalytic sites for the methanol steam reforming Applied Catalysis B. *Environmental*, 2019, vol. 258, 118016
- **A. BIALAS ; T. KONDRATOWICZ ; M. DROZDEK ; P. KUŚTROWSKI**. Catalytic combustion of toluene over copper oxide deposited on twotypes of yttria-stabilized zirconia. *Catalysis Today*, 2015, vol. 257, 144-149
- **T. TSONCHEVA ; I. GENOVA ; M. DIMITROV ; E. SARCADI-PRIBOCZKI ; A. M. VENEZIA ; D. KOVACHEVA ; N. SCOTTI ; V. DAL SANTO**. Nanostructured copper-zirconia composites as catalysts for methanol decomposition. *Applied Catalysis B: Environmental*, 2015, vol. 165, 599-610

- **K. A. ALI ; A. Z. ABDULLAH ; A. R. MOHAMED**. Recent development in catalytic technologies for methanol synthesis from renewable sources: A critical review. *Renewable and Sustainable Energy Reviews*, 2015, vol. 44, 508-518
- **K. LI ; J. G. CHEN**. CO2 Hydrogenation to Methanol over ZrO2-Containing Catalysts: Insights into ZrO2 Induced Synergy. *ACS Catal.*, 2019, vol. 9, 7840-7861
- **P. LIU ; Y. YANG ; M. G. WHITE**. Theoretical perspective of alcohol decomposition and synthesis from CO2 hydrogenation. *Surface Science Reports*, 2013, vol. 68, 233-272
- **K. INUI ; T. KURABAYASHI ; S. SATO**. Direct synthesis of ethyl acetate from ethanol carried out under pressure. *Journal of Catalysis*, 2002, vol. 212, 207-215
- **A. G. SATO ; D. P. VOLANTI ; D. M. MEIRA ; S. DAMYANOVA ; E. LONGO ; J. M. C. BUENO**. Effect of the ZrO2 phase on the structure and behavior of supported Cu catalysts for ethanol conversion. *Journal of Catalysis*, 2013, vol. 307, 1-17
- **GUI-SHENG WU ; DONG-SEN MAO ; GUAN-ZHONG LU ; YONG CAO ; KANG-NIAN FAN**. The Role of the Promoters in Cu Based Catalysts for Methanol Steam Reforming. *Catal Lett*, 2009, vol. 130, 177-184
- **K. INUI ; T. KURABAYASHI ; S. SATO**. Direct synthesis of ethyl acetate from ethanol over Cu-Zn-Zr-Al-O catalyst. *Applied Catalysis A: General*, 2002, vol. 237, 53-61
- **INSOO RO ; YIFEI LIU ; MADELYN R. BALL ; DAVID H. K. JACKSON ; JOSEPH PAUL CHADA ; CANAN SENER ; THOMAS F. KUECH ; ROSTAM J. MADON ; GEORGE W. HUBER ; JAMES A. DUMESIC**. Role of the Cu-ZrO2 Interfacial Sites for Conversion of Ethanol to Ethyl Acetate and Synthesis of Methanol from CO2 and H2. *ACS Catal.*, 2016, vol. 6, 7040-7050
- **H. MIURA ; K. NAKAHARA ; T. KITAJIMA ; T. SHISHIDO**. Concerted Functions of Surface Acid-Base Pairs and Supported Copper Catalysts for Dehydrogenative Synthesis of Esters from Primary Alcohols. *ACS Omega*, 2017, vol. 2, 6167-6173
- **A. G. SATO ; D. P. VOLANTI ; I. C. DE FREITAS ; E. LONGO ; J. M. C. BUENO**. Site-selective ethanol conversion over supported copper catalysts. *Catalysis Communications*, 2012, vol. 26, 122-126
- **K. SAMSON ; M. SLIWA ; R. P. SOCHA ; K. GÓRA-MAREK ; D. MUCHA ; D. RUTKOWSKA-ZBIK ; J-F. PAUL ; M. RUGGIERO-MIKOLAJCZYK ; R. GRABOWSKI ; J. SLOCZYŃSKI**. Influence of ZrO2 Structure and Copper Electronic State on Activity of Cu/ZrO2 Catalysts in Methanol Synthesis from CO2. *ACS Catal.*, 2014, vol. 4, 3730-3741
- **G. PRIETO ; J. ZEČEVIĆ ; H. FRIEDRICH ; K. P. DE JONG ; P. E. DE JONGH**. Towards stable catalysts by controlling collective properties of supported metal nanoparticles. *Nat Mater*, 2013, vol. 12, 34-39
- **J. ZHOU ; Y. ZHANG ; G. WU ; D. MAO ; G. LU**. Influence of the component interaction over Cu/ZrO2 catalysts induced with fractionated precipitation method on the catalytic performance for methanol steam reforming. *RSC Adv.*, 2016, vol. 6, 30176-30183
- **C.Z. YAO ; L.C. WANG ; Y.M. LIU ; G.S. WU ; Y. CAO ; W.L. DAI ; H.Y. HE ; K.N. FAN**. Effect of preparation method on the hydrogen production from methanol steam reforming over binary Cu/ZrO2 catalysts. *Applied Catalysis A: General*, 2006, vol. 297, 151-158
- **R. RAUDASKOSKI ; E. TURPEINEN ; R. LENKKERI ; E. PONGRA' CZ ; R.L. KEISKI**. Catalytic activation of CO2: Use of secondary CO2 for the production of synthesis gas and for methanol synthesis over copper-based zirconia-containing catalysts. *Catalysis Today*, 2009, vol. 144, 318-323